# EUROPEAN PATENT SPECIFICATION

(11) **EP 0 692 534 B1**
(45) Date of publication and mention of the grant of the patent: **09.09.1998**
(21) Application number: 95304165.4
(22) Date of filing: 15.06.1995
(51) Int. Cl.: C12N 15/11, C12N 15/31, C12N 15/80, C12N 15/90, C12P 21/02, C07K 14/37

(54) **A chromosome integration vector**
Chromosomaler Integrationsvektor
Vecteur d'intégration chromosomique

(30) Priority: 29.06.1994 JP 168611/94; 30.03.1995 JP 95831/95; 30.03.1995 JP 95955/95; 17.05.1995 JP 141393/95
(43) Date of publication of application: 17.01.1996
(73) Proprietor: TAKARA SHUZO CO. LTD., Fushimi-ku, Kyoto-shi, Kyoto-fu (JP)
(72) Inventor: Yoshioka, Hirofumi, Kusatsu-shi, Shiga-ken (JP); Oshima, Atsushi, Otsu-shi, Shiga-ken (JP); Takesako, Kazutoh, Otsu-shi, Shiga-ken (JP); Ogawa, Junko, Otsu-shi, Shiga-ken (JP); Okado, Takashi, Soraku-gun, Kyoto-fu (JP); Kato, Ikunoshin, Uji-shi, Kyoto-fu (JP)
(74) Representative: Wakerley, Helen Rachael

(56) References cited:
- EP-A- 0 644 262
- EMBL/GenBank/DDBJ databases (10.05.94) Boyer, J. et al. "S. cerevisiae chromosome XI reading frame ORF YKL004w" XP002011025
- EMBL/GenBank/DDBJ databanks (01.06.94) Boyer, J. et al. "Hypothetical 45.2KD protein RPL14A-MRP17 intergenic region" XP002011027
- J ANTIBIOT (TOKYO), SEP 1993, 46 (9) P1347-54, JAPAN, XP002010973 YOSHIKAWA Y ET AL: "Isolation, structures, and antifungal activities of new *aureobasidins*."

## Description

This invention relates to a vector capable of imparting a novel dominant genetic marker to a host, which can be integrated into the chromosome of fungal cells and by which a transformant can be easily detected from these cells inoculated into a specific medium, a process for producing a transformant having a marker imparted thereto with the use of the above mentioned vector, and a transformant transformed by this vector.

The present invention further relates to a mutant of a protein capable of imparting the sensitivity to an antimycotic aureobasidin, a mutant mutated into an aureobasidin resistant protein, and a DNA coding for this protein.

There have been known techniques for introducing useful genes into monoploid fungal cells to be used in a laboratory, for example, Saccharomyces cerevisiae (hereinafter referred to simply as S. cerevisiae), Schizosaccharomyces pombe (hereinafter referred to simply as Schizo. pombe) and Aspergillus nidulans (hereinafter referred to simply as A. nidulans). Since the incorporation and fixation of plasmid DNAs into fungal cells are relatively scarcely successful, it is required to use selective markers in the identification of transformants. In the most common case, selection can be achieved by introducing an auxotrophic mutation into host cells. Examples of the mutation generally employed in, for example, S. cerevisiae include ura3, leu2, trp1 and his3. A plasmid carries a wild type copy of one of these genes. Since the wild type copy on the plasmid is dominant over the chromosomal allele of the host, cells having the plasmid introduced thereinto can be screened in a minimal medium which contains no nutrient required by the auxotrophic host cells. Also there have been published some reports, though in a small number, relating to the use of drug resistance in the screening of transformants. Namely, there have been reported replication vectors and chromosome integration vectors containing genes which are resistant against antibiotics such as a neomycin homologue G418, hygromycin and cerulenin. A replication vector has a DNA replication origin acting in a cell. This plasmid is held outside the chromosome as a cyclic episome and continuously reduced at a ratio of several percent with the proliferation of the cells. An integration vector is inserted into the chromosome of a host cell and thus held in a stable state. In this case, therefore, it is unnecessary to further add a drug to the medium in order to exert the selection function for maintaining the sequence of the vector.

In the case of industrial fungi, it is required to sustain the useful character, which has been imparted thereto, in a stable state. A chromosome integration vector is useful for this purpose.

Fungi have been widely applied to liquors such as sake, beer and wine and fermented foods such as miso (fermented soy bean paste) and soy sauce. For breeding these fungi to be used for industrial purposes, genetic engineering techniques are also highly effective in order to impart useful characteristics thereto. Thus there have been required selective markers which are usable in efficiently screening transformants. Industrial yeasts are usually di- or polyploid cells. It is therefore difficult to introduce an auxotrophic marker, which is effective in monoploid cells of, for example, yeasts to be used in a laboratory, into these industrial yeasts. In addition, since there is a high possibility that a mutagenesis induces mutation in other genes, accordingly, it is highly difficult to create a mutant having the desired auxotrophic mutation alone introduced thereinto. The use of a drug resistance makes it possible to screen a stable transformant of an arbitrary yeast regardless of the number of chromosomes or the occurrence of specific mutation. However many of these industrial fungi are insensitive to antibiotics such as G-418 and hygromycin, which makes it impossible to use genes resistant against these antibiotics therefor. Moreover, these resistant genes are genes or proteins derived from bacteria which are procaryotes, and none of them corresponding to these genes is present in fungi such as yeasts. The use of fungal cells having these foreign genes integrated therein is seriously restricted. A cerulenin resistant gene (PDR4) originating in S. cerevisiae is usable in the transformation of S. cerevisiae including brewing yeast. However it also conferred resistances against drugs other than cerulenin, which might bring about some problems in the practical use. Therefore PDR4 cannot fully satisfy the requirements for breeding industrial fungi including S. cerevisiae having improved characters in future. Thus it has been required to develop drug resistant markers with the use of genes which are inherently carried by fungi.

Under these circumstances, the present invention aims at providing a novel chromosome integration vector capable of imparting a novel selective marker of a drug resistance to a fungal transformant, and a transformant transformed by this vector.

The present invention further aims at providing a protein capable of imparting the aureobasidin resistance and acting as a selective marker which is usable in genetic engineering of fungi, and a DNA coding for this protein.

The present invention may be summarized as follows. Namely, the first aspect of the present invention relates to a chromosome integration vector for a host fungus which is characterized by containing an aureobasidin resistant gene. This chromosome integration vector sometimes contains a foreign gene. The second aspect of the invention relates to a process for producing an aureobasidin resistant transformant characterized by comprising:
(1) the step of obtaining a replication vector which contains an aureobasidin resistant gene,
(2) the step of cleaving the aureobasidin resistant gene in the replication vector obtained in the above step at one site to give a chromosome integration vector for a host fungus;
(3) the step of integrating the chromosome integration vector for a host fungus obtained in the above step into the chromosome of the host fungus; and
(4) the step of selecting a host which has been transformed into an aureobasidin resistant one in the presence of aureobasidin.

In this process for producing an aureobasidin resistant transformant, the replication vector sometimes contains a foreign gene. The third aspect of the invention relates to a transformant characterized by being one obtained by the process of the second aspect of the invention.

The fourth aspect of the invention relates to a protein capable of imparting aureobasidin resistance, wherein at least the 240th amino acid residue Ala in the protein capable of imparting aureobasidin sensitivity represented by SEQ ID No. 8 in the Sequence Listing has been replaced by another amino acid residue, or another protein capable of imparting aureobasidin resistance which has an amino acid sequence obtained by subjecting the above-mentioned protein to at least one modification selected from replacement, insertion and deletion of amino acid residue(s) and shows a biological activity comparable to that of the above-mentioned protein. The fifth aspect of the invention relates to a DNA which codes for the protein capable of imparting the aureobasidin resistance of the fourth aspect of the invention.

Aureobasidin [Japanese Patent Laid-Open No. 138296/1990, No. 22995/1991, No. 220199/1991 and No. 279384/1993, Japanese Patent Application No. 303177/1992, J. Antibiotics, 44 (9), 919 - 924, ibid., 44 (9), 925 -933, ibid., 44 (11), 1187 - 1198 (1991)] is a cyclic depsipeptide obtained as a fermentation product of a strain Aureobasidium pullulans No. R106 (FERM BP-1938). It is completely different in structure from other antimycotics. As Tables 1 and 2 show, aureobasidin A, which is a typical aureobasidin compound, exerts a potent antimycotic activity on various yeasts of the genus Candida including Candida albicans (hereinafter referred to simply as C. albicans Cryptococcus neoformans, Histoplasma capsulatum, Blastomyces dermatitidis and fungi of the genus Aspergillus (Japanese Patent Laid-Open No. 138296/1990) but has an extremely low toxicity in mammal. Thus this compound is an antimycotic being excellent in selective toxicity.

Hereinafter, Candida, Cryptococcus and Aspergillus will be abbreviated respectively as C., Cr. and A.

**[Table 1]**

| Test strain | TIMM No. | MIC(µg/ml) |
|---|---|---|
| C. albicans | 0136 | ≦0.04 |
| C. albicans var. stellatoidea | 1308 | ≦0.04 |
| C. tropicalis | 0312 | 0.08 |
| C. kefyr | 0298 | 0.16 |
| C. parapsilosis | 0287 | 0.16 |
| C. krusei | 0270 | ≦0.04 |
| C. guilliermondii | 0257 | 0.08 |
| C. glabrata | 1062 | ≦0.04 |
| Cr. neoformans | 0354 | 0.63 |
| Cr. terreus | 0424 | 0.31 |
| Rhodotorula rubra | 0923 | 0.63 |
| A. fumigatus | 0063 | 20 |
| A. clavatus | 0056 | 0.16 |

**[Table 2]**

| Test strain | TIMM No. | MIC (µg/ml) |
|---|---|---|
| A. nidulans | 0112 | 0.16 |
| A. terreus | 0120 | 5 |
| Penicillium commune | 1331 | 1.25 |
| Trichophyton mentagrophytes | 1189 | 10 |
| Epidermophyton floccosum | 0431 | 2.5 |
| Fonsecaea pedrosoi | 0482 | 0.31 |
| Exophiala werneckii | 1334 | 1.25 |
| Cladosporium bantianum | 0343 | 0.63 |
| Histoplasma capsulatum | 0713 | 0.16 |
| Paraccoccidioides brasiliensis | 0880 | 0.31 |
| Geotrichum candidum | 0694 | 0.63 |
| Blastomyces dermatitidis | 0126 | 0.31 |

As described in Japanese Patent Application No.106158/1994, the present inventors have previously found out that fungi such as Schizo. pombe and S. cerevisiae show a sensitivity to aureobasidin.

**[Table 3]**

| Test strain or cell | MIC(µg/ml) |
|---|---|
| Schizo. pombe | 0.08 |
| S. cerevisiae | 0.31 |

The present inventors have mutagenized a wild-type strain of Schizo. pombe or S. cerevisiae, sensitive to aureobasidin, to thereby give resistant mutants. We have further successfully isolated a gene capable of confering aureobasidin resistance (a resistant gene) from these resistant mutants and another gene capable of imparting aureobasidin sensitivity (a sensitive gene) from the corresponding sensitive cells. Furthermore, We have disclosed the existence of a protein encoded by each of these genes. The present inventors have also succeeded in the expression of this gene by preparing e replication vector containing this gene and incubating cells transformed by using this vector. By using a DNA fragment of this gene as a probe, they have further successfully found a novel gene regulating the aureobasidin sensitivity from another fungus which is sensitive to aureobasidin.

The fungi having an aureobasidin sensitivity listed in Tables 1 and 2 and the fungi listed in Table 3 each carries a protein regulating the aureobasidin sensitivity and a gene coding for this protein. The term "a protein regulating aureobasidin sensitivity" as used herein means a protein which is contained in a fungus having a sensitivity to aureobasidin. This protein is required for the expression of the sensitivity or resistance to aureobasidin. As a matter of course, a protein having 35% or more homology with the above-mentioned protein and having a similar function is also a member of the protein regulating aureobasidin sensitivity. Furthermore, proteins obtained by modifying these proteins by the genetic engineering procedure are members of the protein regulating aureobasidin sensitivity. A gene regulating aureobasidin sensitivity means a gene which codes for such a protein regulating aureobasidin sensitivity as those described above and involves both of sensitive genes and resistant genes.

To isolate the gene regulating the aureobasidin sensitivity, aureobasidin sensitive cells (a wild strain) are first subjected to a mutagenesis to thereby derive a resistant strain therefrom. Next, a DNA library is prepared from the chromosome DNAs or cDNAs of this resistant strain and a gene capable of imparting the resistance (resistant gene) is cloned from this library. Also, a sensitive gene can be isolated by preparing a DNA library of the wild strain, isolating DNA molecules hybridizable with the resistant gene from this library and cloning the same.

The mutagenesis is performed by, for example, treating with a chemical such as ethylmethane sulfonate (EMS) or N-methyl-N'-nitro-N-nitrosoguanidine (MNNG) or by ultraviolet or other radiation. The cell that has acquired the resistance can be screened by culturing the mutagenized cells in a nutritional medium containing aureobasidin at an appropriate concentration under appropriate conditions. The resistant strain thus obtained may vary depending on the method and conditions selected for the mutagenesis.

As typical examples of the genes regulating aureobasidin sensitivity(named aur) according to the present invention, aurl genes may be cited. Typical examples of the aurl gene include spaur1 gene isolated from Schizo. pombe and scaurl gene isolated from S. cerevisiae. Now, resistant genes (spaur1^{R} and scaur1^{R}) isolated from resistant mutants by the present inventors and sensitive genes (spaur1^{S} and scaur1^{S}) isolated from sensitive wild-type strains will be described.

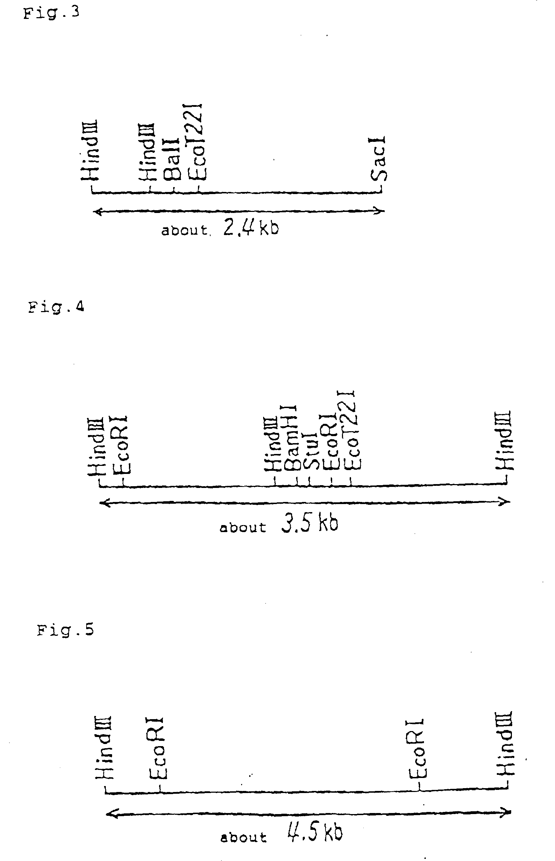

Fig. 3 shows a restriction enzyme map of the genes spaur1^{R} and spaur1^{S} regulating aureobasidin sensitivity, Fig. 4 shows a restriction enzyme map of scaur1^{R} and scaur1^{S}.

Schizo. pombe, which is sensitive to aureobasidin, is mutagenized with EMS and a genomic library of the resistant strain thus obtained is prepared. From this library, a DNA fragment containing a resistant gene (spaur1^{R}) and having the restriction enzyme map of Fig. 3 is isolated. This gene has a nucleotide sequence represented by SEQ ID No. 1 in the Sequence Listing. The amino acid sequence of a protein encoded by this gene, which is estimated on the basis of this nucleotide sequence, is the one represented by SEQ ID No. 2 in the Sequence Listing. By the hybridization with the use of this resistant gene as a probe, a DNA fragment containing a sensitive gene (spaur1^{s}) and having the restriction enzyme map of Fig. 3 is isolated from a sensitive strain. This gene has a nucleotide sequence represented by SEQ ID No. 3 in the Sequence Listing. The amino acid sequence of a protein encoded by this gene, which is estimated on the basis of this nucleotide sequence, is the one represented by SEQ ID No. 4 in the Sequence Listing. A comparison between the sequences of SEQ ID No. 3 and SEQ ID No. 1 reveals that a mutation from G to T occurs at the base at the position 1053, while a comparison between the sequences of SEQ ID No. 4 and SEQ ID No. 2 reveals that glycine at the residue 240 is converted into cysteine at the amino acid level, thus giving rise to the resistance.

Also, S. cerevisiae, which is sensitive to aureobasidin, is mutagenized with EMS and genomic libraries of a resistant strain thus obtained are prepared. A DNA fragment containing a resistant gene (scaur1^{R}) as a dominant mutant and having the restriction enzyme map of Fig. 4 is isolated.

The nucleotide sequence of the coding region for the protein of the scaur1^{R} gene is the one represented by SEQ ID No. 5 in the Sequence Listing. The amino acid sequence of the protein encoded by this gene, which is estimated on the basis of the above nucleotide sequence, is the one represented by SEQ ID No. 6 in the Sequence Listing. By the hybridization with the use of this resistant gene scaur1^{R} as a probe, a DNA fragment containing a sensitive gene (scaur1^{S}) and having the restriction enzyme map of Fig. 4 is isolated from a sensitive strain. This gene has a nucleotide sequence represented by SEQ ID No. 7 in the Sequence Listing. The amino acid sequence of a protein encoded by this gene, which is estimated on the basis of this nucleotide sequence, is the one represented by SEQ ID No. 8 in the Sequence Listing. A comparison between the sequences of SEQ ID No. 7 and SEQ ID No. 5 reveals that a mutation from T to A occurs at the base at the position 852, while a comparison between the sequences of SEQ ID No. 8 and SEQ ID No. 6 reveals that phenylalanine at the residue 158 is converted into tyrosine at the amino acid level, thus giving rise to the resistance. The spaur1 gene has a 58% homology with the scaurl gene at the amino acid level. Thus it is obvious that they are genes coding for proteins having similar functions to each other. When genes and proteins being homologous in sequence with the spaur1 and scaur1 genes and with the proteins encoded thereby are searched from a data base, none having a homology of 35% or above is detected. Accordingly, it is clear that these genes and the proteins encoded thereby are novel molecules which have never been known hitherto.

Next, a pair of primers represented by SEQ ID Nos. 9 and 10 in the Sequence Listing are synthesized, on the basis of the DNA base sequence of the spaur1^{R} gene represented by SEQ ID No. 1. By using these primers, PCR is effected with the use of the cDNA of a pathogenic fungi C. albicans as a template. The PCR product thus obtained is electrophoresed on an agarose gel and stained with ethidium bromide to thereby confirm the specific amplification of the DNA fragment.

By using the DNA fragment thus obtained as a probe, the genomic DNA library of C. albicans is screened. Thus a DNA molecule containing a gene (caaur1), which has a function similar to those of the spaur1 and scaur1 genes, and having a restriction enzyme map of Fig. 5 is obtained. The base sequence of this caaur1 gene is one represented by SEQ ID No. 11 in the Sequence Listing. The amino acid sequence of the protein coded for by this gene estimated on the basis of this base sequence is one represented by SEQ ID No. 12 in the Sequence Listing. Thus this protein has a high homology with the proteins coded for by the spaur1 and scaur1 genes.

As the above-mentioned examples clearly show, a gene regulating the aureobasidin sensitivity corresponding to each organism or each method can be isolated by employing a starting material, which is an organism having the sensitivity to aureobasidin, and effecting cloning by conducting various mutagenesis and/or screening treatments in the same manner as the one described above. Moreover, genes hybridizable with these genes can be isolated. As a matter of course, it is possible to prepare modified genes by partly altering the genes regulating the aureobasidin sensitivity obtained above by chemical, physical or genetic engineering techniques.

In the present invention, an aureobasidin resistant gene refers to a gene which is capable of imparting the resistance to an antimycotic aureobasidin when integrated into a host fungus. This gene codes for a protein imparting an aureobasidin resistance.

The aureobasidin resistant gene is exemplified typically by the above-mentioned spaur1^{R} and scaur1^{R}. Such a gene acts dominantly and the resistance conferred by this gene is selective to aureobasidin. That is to say, it does not cause any substantial change in the sensitivity to other drugs.

The aureobasidin resistant gene also involves genes which are hybridizable with spaur1^{R} and scaur1^{R} and impart the aureobasidin resistance to a host fungus (for example, genes prepared by partly altering the spaur1^{R} or scaur1^{R} gene by chemical, enzymatic, physical or genetic engineering techniques).

Furthermore, the aureobasidin resistant gene involves a gene coding for a protein, which has an amino acid sequence obtained by subjecting a protein (Aur1^{R}p) capable of imparting the aureobasidin resistance to at least one modification selected from replacement, insertion and deletion of amino acid residue(s) and shows the activity of imparting the aureobasidin resistance.

The replacement, insertion and deletion of amino acid residue(s) from Aur1^{R}p can be effected by a site-specific mutagenesis. A DNA coding for the isolated Aur1^{R}p or a DNA coding for the protein capable of imparting the aureobasidin sensitivity (Aur1^{S}p) can be easily modified by effecting at least one of the replacement, insertion and deletion of nucleotide(s) and thus a novel DNA coding for a mutant of Aur1^{R}p can be obtained. Regarding the replacement, insertion and deletion of amino acid residue(s) the conversion of the amino acid(s) is based on one which can be effected by genetic engineering techniques without deteriorating the biological activity. In order to appropriately effect the mutation on the residue at a specific site, the target codon is subjected to random mutagenesis and a mutant having the desired activity is screened from the ones thus expressed. The mutant obtained by insertion involves a fused protein wherein Aur1^{R}p or its fragment is bound to another protein or polypeptide at the amino terminal and/or the carboxyl terminal of the Aur1^{R}p or its fragment via a peptide bond. In order to delete amino acid residue(s), it is also possible to replace an arbitrary amino acid codon in the amino acid sequence with a termination codon by the gapped duplex method to thereby delete the region on the carboxyl terminal side of the replaced amino acid residue from the amino acid sequence. Alternatively, a DNA coding for a protein, from which the amino terminal and/or carboxyl terminal regions in an arbitrary length have been deleted, can be obtained by the deletion method comprising degrading the coding DNA from the region(s) corresponding to the amino terminal and/or the carboxyl terminal of the amino acid sequence [Gene, 33, 103 - 119 (1985)] or a PCR method with the use of primers containing an initiation codon and/or a termination codon. Known examples of the site-specific mutagenesis method include the gapped duplex method with the use of oligonucleotide(s) [Methods in Enzymology, 154, 350 - 367 (1987)], the uracil DNA method with the use of oligonucleotide(s) [Methods in Enzymology, 154, 367 - 382 (1987)], the nitrous acid mutation method [Proc. Natl. Acad. Sci. USA, 79, 7258 - 7262 (1982)] and the cassette mutation method [Gene, 34, 315 - 323 (1985)].

The present inventors have found out that Aur1^{s}p represented by SEQ ID No. 8 in the Sequence Listing can be converted into Aur1^{R}p by replacing the 240th residue Ala by another amino acid residue, thus completing the fourth and fifth aspects of the present invention.

The Aur1^{R}p of the fourth aspect of the invention is one wherein the 240th residue Ala of Aur1^{S}p represented by SEQ ID No. 8 in the Sequence Listing has been replaced by another amino acid residue. Other amino acid residues may be replaced, inserted or deleted by using chemical, physical or genetic engineering techniques, so long as the biological activity is not deteriorated thereby. The Aur1^{R}p of the fourth aspect of the invention may be appropriately prepared through genetic engineering techniques by using a DNA coding for Aur1^{S}p represented by SEQ ID No. 23 in the Sequence Listing. Its biological activity can be assayed by measuring the activity of converting aureobasidin sensitive cells into resistant cells. The Aur1^{R}p of the fourth aspect of the invention is one having an enhanced activity of converting aureobasidin sensitive cells into resistant cells compared with Aur1^{R}p represented by SEQ ID No. 6 in the Sequence Listing.

A preferable Aur1^{R}p is one having an enhanced activity of converting aureobasidin sensitive cells into resistant cells compared with Aur1^{R}p represented by SEQ ID No. 6 in the Sequence Listing. A DNA coding for this Aur1^{R}p can be appropriately used in the present invention.

In an example of particularly preferable embodiment of Aur1^{R}p, a mutant can be obtained by replacing the 240th residue Ala by Cys. The amino acid sequence of an example of such a mutant is shown in SEQ ID No. 18 in the Sequence Listing. This mutant is referred to as Aur1^{R}p (A240C). It is also possible to obtain a mutant wherein the 158th residue Phe and the 240th residue Ala of Aur1^{S}p have been replaced respectively by Tyr and Cys. The amino acid sequence of this mutant is shown in SEQ ID No. 19 in the Sequence Listing. This mutant is referred to as Aur1^{R}p (F158Y, A240C). Each of these mutants has a stronger ability to impart aureobasidin resistance than that of the protein represented by SEQ ID No. 16 in the Sequence Listing [Aur1^{R}p (F158Y)] wherein the 158th residue Phe of Aur1^{S}p has been replaced by Tyr.

The aureobasidin resistant gene to be used in the present invention is exemplified by the DNAs represented by SEQ ID Nos. 20 to 22 in the Sequence Listing. The DNA represented by SEQ ID No. 22 in the Sequence Listing is one coding for Aur1^{R}p (F158Y), the DNA represented by SEQ ID No. 20 in the Sequence Listing is one coding for Aur1^{R}p (A240C), and the DNA represented by SEQ ID No. 21 in the Sequence Listing is one coding for Aur1^{R}p (F158Y, A240C).

A replication plasmid can be prepared by integrating a gene, which coded for a protein regulating the aureobasidin sensitivity, into an appropriate vector. For example, a plasmid prepared by integrating an aureobasidin resistant gene into an appropriated yeast vector is highly useful as a selective marker gene, since a transformant can be easily selected thereby depending on the drug resistance with the use of aureobasidin. As the vector for yeasts, use can be made of ones of YRₚ, YCₚ, YEₚ and YIₚ types.

Also, the replication plasmid can be stably carried by, for example, Escherichia coli. Examples of vectors which are usable in this case include pUC118, pWH5, pAU-PS, Traplex119 and pTV118. pAU-PS having the scaur1^{S} gene integrated therein is named pSPAR1. pWH5 having the spaur1^{^{S}} gene integrated therein is named pSCAR1. Traplex119 vector having the caaur1 gene integrated therein is named pCAAR1. Each of these recombinant plasmids is transformed into E. coli. It is also possible to express these plasmids in an appropriate host. Such a gene is reduced exclusively into the open reading frame (ORF) to be translated into a protein by cleaving with an appropriate restriction enzyme, if necessary, and then bound to an appropriate vector. Thus an expression recombinant plasmid can be obtained. When E. coli is used as the host, plasmids such as pTV118 may be used as a vector for the expression plasmid. When a yeast is used as the host, plasmids such as pYES2 may be used as the vector. E. coli JM109 transformed by pSPAR1 having the spaur1^{S} gene integrated therein has been named and designated as Escherichia coli 1M109/pSPAR1 and deposited at National Institute of Bioscience and Human-Technology, Agency of Industrial Science and Technology (1-3, Higashi 1 chome Tsukuba-shi Ibaraki-ken 305, JAPAN), in accordance with the Budapest Treaty under the accession number FERM BP-4485. E. coli HB101 transformed by pSCAR1 having the scaur1^{S} gene integrated therein has been named and designated as Escherichia coli HB101/pSCAr1 and deposited at National Institute of Bioscience and Human-Technology, Agency of Industrial Science and Technology in accordance with the Budapest Treaty under the accession number FERM BP-4483. E. coli HB101 transformed by pCCAR1 having the caaur1^{s} gene integrated therein has been named and designated as Escherichia coli HB101/pCAAR1 and deposited at National Institute of Bioscience and Human-Technology, Agency of Industrial Science and Technology in accordance with the Budapest Treaty under the accession number FERM BP-4482.

The integration vector containing the aureobasidin resistant gene of the present invention is a linear vector which can be usually prepared by cleaving a replication plasmid containing the aureobasidin resistant gene into a linear form. The cleavage point in the replication plasmid will be described hereinbelow.

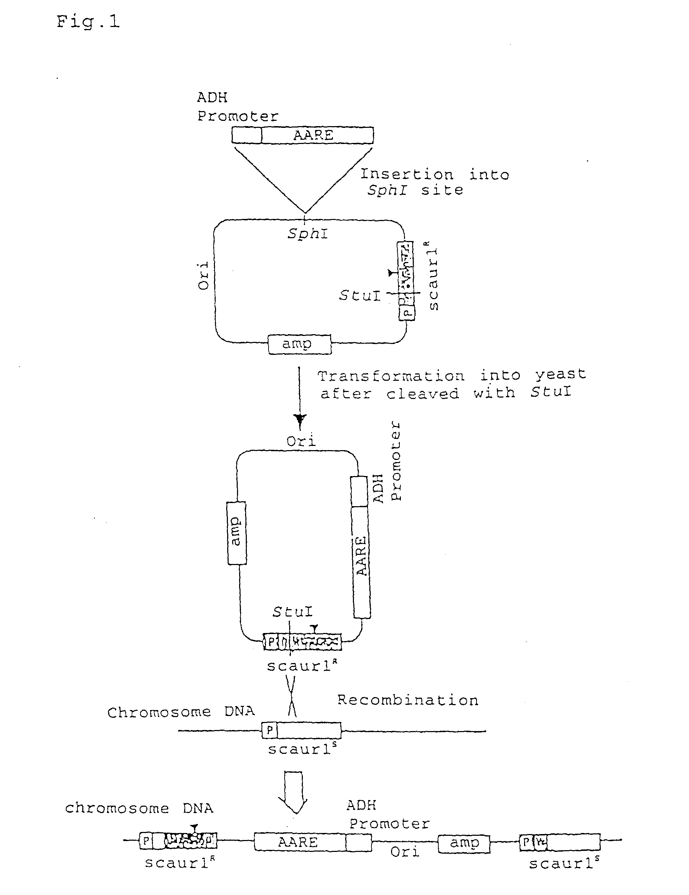

Fig. 1 shows a process wherein an aureobasidin resistant gene in a chromosome integration vector undergoes homologous recombination with the host chromosome being homologous therewith (i.e., an aureobasidin sensitive gene) and thus aureobasidin sensitive cells are converted into aureobasidin resistant cells. A replication plasmid containing the aureobasidin resistant gene is cleaved into a linear form at one position in the aureobasidin resistant gene sequence with an appropriate restriction enzyme. The vector thus linearized undergoes homologous recombination with the aureobasidin sensitive gene in the host chromosome being homologous therewith. Thus the aureobasidin resistance is imparted to the host cells. When the replication plasmid contains a foreign gene, then the aureobasidin resistance and the foreign gene are imparted to the host cells. For example, a replication vector pAuR1aare for preparing a linear vector, which contains scaur1^{R} and human acylamino acid releasing enzyme (AARE) described in Japanese Patent Laid-Open No. 254680/1991, is prepared. Escherichia coli JM109 strain having this vector introduced therein was named and indicated as Escherichia coli JM109/pAUR1aare.

To linearize a replication vector, use can be effectively made of a restriction enzyme cleavage site which exists not in the target foreign gene moiety but in the aureobasidin resistant gene. In the cases of, for example, scaur1^{R} originating in S. cerevisiae and spaur1^{R} originating in S. pombe, restriction enzyme sites of StuI, etc. and BalI, etc. are usable respectively.

In a preferable form, the vector of the present invention may contain an aureobasidin resistant gene and a foreign gene and other genes originating in replication vectors may be eliminated therefrom. Promoters, terminators, etc. for expressing the aureobasidin resistant gene and the foreign gene may be selected depending on the characters of the host. As a matter of course, the promoter parts of the DNAs represented by SEQ ID Nos. 1 and 5 in the Sequence Listing can be used as a promoter for expressing the function of the aureobasidin resistant gene. In the case of S. cerevisiae, use can be made of, for example, promoters of alcohol dehydrogenase gene (ADH1) and glyceraldehyde-3-phosphate dehydrogenase gene (GPD) and the terminator of cytochrome C1 gene (CYC1). These promoters and terminators may be different from those for expressing the aureobasidin resistant gene.

In the present invention, the term "foreign gene" refers to a gene which is foreign to the host fungal cells, i.e., an alien gene. Examples thereof include a nonfungal gene, a modified gene, a gene of a fungal species different from the host and a self-cloned gene. More particularly, genes participating in fermentation, alcohol resistance, saccharification and the formation of taste components or aroma components fall within this category.

The second aspect of the invention relates to a process for producing an aureobasidin resistant transformant. An aureobasidin resistant transformant can be created by, for example, preparing a replication vector containing the above-mentioned aureobasidin resistant gene, cleaving it at one position in the aureobasidin resistant gene in the replication vector to give a linear chromosome integration vector for a host fungus, adding this vector to aureobasidin sensitive host fungal cells under such conditions as to allow the transformation of the fungal cells, thus integrating the vector into the host chromosome, incubating the transformant in a medium suitable for the proliferation of the host cells containing the antibiotic aureobasidin, and screening the aureobasidin resistant transformant thus proliferating. The transformation may be effected in accordance with publicly known methods such as the protoplast generation procedure, the lithium acetateprocedureor the electroporationprocedure. The medium to be used herein is not particularly restricted, so long as it is usable in the proliferation of fungi. Examples of such a medium commonly employed include Sabouraud's dextrose medium, a YPD medium, a czapek medium and a YNBG medium. The concentration of the aureobasidin added varies depending on the host fungal cells having the sensitivity and usually ranges from 0.05 to 80 µg/ml.

The transformant of the third aspect of the invention can be obtained by the process of the second aspect of the invention.

As an example of the transformant according to the present invention, Sake yeast Kyokai K-701 having scaur1^{R} and AARE gene integrated into the chromosome has been obtained.

The transformant thus obtained by using the chromosome integration vector of the present invention has an aureobasidin resistance imparted thereto and the foreign gene integrated thereinto which is held on the chromosome in a stable state. These characteristics make it highly useful in industrial uses, etc.

The Aur1^{R}p of the fourth aspect of the invention can impart the aureobasidin resistance to monoploid yeasts and diploid yeasts, in particular, practically usable ones. Namely, it is highly useful in breeding S. cerevisiae which has been widely applied to liquors such as sake, shochu, beer and wine and fermented foods such as bread. Further, the Aur1^{R}p of the fourth aspect of the invention is applicable to fungi other than S. cerevisiae and useful in, for example, breeding and genetic engineering application of other fungi.

For example, the Aur1^{R}p of the fourth aspect of the invention is capable of imparting the aureobasidin resistance to C. albicans. A vector having the DNA coding for this Aur1^{R}p is the first vector for genetic engineering uses provided for C. albicans.

It is known that C. albicans is a fungus causative of mycosis. With the recent increase in opportunistic infection, it has been needed to conduct studies for clarifying the causes of the pathogenicity. The Aur1^{R}p of the fourth aspect of the invention and the above-mentioned vector are highly useful in genetic studies on C. albicans.

### [Brief Description of the Drawing]

[Fig. 1]
   Construction of the vector which is a example of the present invention, and integration of the vector into the chromosome.
[Fig.2]
   Pattern of the southern hybridization after electrophoresis of the genomic DNA digested by a restriction enzyme.
[Fig.3]
   Restriction enzyme map of the genes spaur1^{R} and spaur1^{S}.
[Fig.4]
   Restriction enzyme map of the genes scaur1^{R} and scaur1^{S}.
[Fig.5]
   Restriction enzyme map of the genes caaur1.

### [Examples]

To further illustrate the present invention in greater detail, the following Examples will be given. However it is to be understood that the present invention is not restricted thereto.

### Example 1: Construction of chromosome integration vector containing aureobasidin resistant gene

### (1) Construction of replication vector containing scaur1^{R}

A plasmid pSCAR1 was prepared from Escherichia coli HB101/pSCAR1 (FERM BP-4483) which carried a plasmid pSCAR1 containing scaur1^{S}. Then the obtained plasmid was partially cleaved with HindIII and thus a DNA of 3.5 kb containing scaur1^{S} was separated therefrom. This DNA (3.5 kb) was ligated to a vector pUC118 cleaved with HindIII to thereby prepare a plasmid pUscaur1^{S}. This plasmid pUscaur1^{S} was transformed into Escherichia coli CJ236 to thereby prepare ssDNA.

Next, a site-specific DNA mutation was introduced by using Mutan-K kit (manufactured by Takara Shuzo Co., Ltd.) with the use of a synthetic oligonucleotide for introducing mutation represented by SEQ ID No. 13 in the Sequence Listing, which had been synthesized and purified, and the above-mentioned ssDNA. That is to say, the use of the oligonucleotide represented by SEQ ID No. 13 in the Sequence Listing made it possible to obtain scaur1^{R} wherein the codon TTT of the 158th amino acid residue Phe in the ORF of the gene scaur1^{S} had been replaced by the codon TAT of Tyr. This plasmid having a DNA coding for Aur1^{R}p (F158Y) was designated as pUscaur1R.

### (2) Amplification of scaur1^{R} by PCR method

By using the plasmid pUscaur1R which carried a HindIII fragment of 3.5 kb containing scaur1^{R}, scaur1^{R} (about 1.9 kb) was amplified by the PCR method. Regarding primers employed herein, XhoI and KpnI sites had been designed in primers in order to clone the amplified scaur1^{R} into the plasmid vector pYES2 (manufactured by Invitrogen corporation) and thus primers represented by SEQ ID Nos. 14 and 15 in the Sequence Listing were synthesized.

The reaction was effected in the following manner. 100 µl of a PCR solution containing 28 µl of a PCR buffer [capable of giving final concentrations of 10 mM of Tris-HCl (pH 8.3), 50 mM of KCl, 1.5 mM of MgCl₂, 0.1 mM of dATP, 0.1 mM of dCTP, 0.1 mM of dTTP and 0.1 mM of dGTP], 1 µl of 2.5 U of Ampli Taq DNA polymerase (Manufactured by Perkin-Elmer), 0.5 µl portions of 20 pmol of the primers represented by SEQ ID Nos. 14 and 15 in the Sequence Listing, 1 µl of the plasmid and 69 µl of H₂O was maintained at an initial temperature of 94°C for 1 minute, and then heated successively at 94°C for 1 minute, at 50°C for 2 minutes and at 72°C for 3 minutes. This heating cycle was repeated 35 times. Next, the reaction mixture was maintained at 72°C for 10 minutes to thereby effect the amplification by PCR. Then the PCR amplification product was cleaved with KpnI and XhoI and electrophoresed on an agarose gel and the target DNA fragment of about 1.9 kb was recovered from the gel and purified by using Suprec™-01 (manufactured by Takara Shuzo Co., Ltd.).

### (3) DNA ligation and transformation

About 0.3 µg of the DNA fragment (about 1.9 kb) purified in the above step was ligated to about 0.1 µg of pYES2, which had been digested with XhoI and KpnI, by using a DNA ligation kit (manufactured by Takara Shuzo Co., Ltd.).

Next, 7 µl of the above-mentioned ligation mixture was added to 200 µl of competent cells of Escherichia coli HB101. These cells were allowed to stand on ice for 30 minutes, at 42°C for 1 minute and then on ice again for 1 minute. Then 800 µl of an SOC medium [Sambrook et al., "Molecular Cloning, A Laboratory Manual", Cold Spring Harbor Laboratory Press (1989)] was added thereto. After incubating at 37°C for 1 hour, these Escherichia coli cells were spread onto an L-broth agar medium containing 50 µg/ml of ampicillin and incubated at 37°C overnight. Thus a transformant was obtained.

This transformant was incubated in 5 ml of an L-broth medium containing 50 µg/ml of ampicillin at 37°C overnight. From this culture, a plasmid DNA was prepared in accordance with the alkali method ("Molecular Cloning", cited above). The plasmid thus obtained was named pYES2aur1.

### (4) Construction of chromosome integration vector

About 0.4 µg of the above-mentioned plasmid pYES2aur1 was digested with Xbal and KpnI and electrophoresed on an agarose gel. Then a DNA fragment of about 1.9 kb containing scaur1^{R} was recovered from the gel and purified by using Suprec-O1.

Similarly, about 0.4 µg of the plasmid vector pUC19 was digested with SspI and PvuII and electrophoresed on an agarose gel. Then a DNA fragment (about 1.8 kb) containing an ampicillin resistant gene and ColE1 origin was recovered from the gel and purified. About 0.1 µg portions of these DNA fragments thus purified were blunt-ended with the use of a DNA blunting kit (manufactured by Takara Shuzo Co., Ltd.). Further, about 0.1 µg portions of these blunted DNA fragments were subjected to a ligation reaction. The ligation reaction was effected by using a DNA ligation kit (manufactured by Takara Shuzo Co.,Ltd.).

Subsequently, the plasmid was integrated into Escherichia coli JM 109. After incubating, a transformant and a plasmid DNA were prepared. The plasmid thus obtained was named plasmid pAUR1. Next, this plasmid was cleaved with StuI to thereby prepare a chromosome integration vector.

### Example 2: Construction of chromosome integration vector containing aureobasidin resistant gene

### (1) Amplification of DNA fragment of scaur1^{R} having mutation introduced therein by PCR

In order to replace the 240th amino acid residue Ala of Aur1^{R}p (F158Y) by Cys, a primer, wherein the codon GCT of Ala had been changed into the codon TGT of Cys, represented by SEQ ID No. 24 in the Sequence Listing was synthesized and purified. By using the plasmid pUscaurlR described in Example 1-(1), which carried a HindIII fragment (3.5 kb) containing scaur¹R at the HindIII site of pUC119, as a template, was amplified a DNA fragment (about 1.4 kb) which contained a sequence of about 500 bp coding for the amino acid sequence on the C-terminal side of Aur1^{R}p (F158Y, A240C), wherein GCT had been changed into TGT, by the PCR method with the use of the primer represented by SEQ ID No. 24 in the Sequence Listing and a primer M13M4. The PCR was effected in the following manner. To 28 µl of a PCR buffer [capable of giving final concentrations of 10 mM of Tris-HCl (pH 8.3), 50 mM of KCl, 1.5 mM of MgCl₂, 0.1 mM of dATP, 0.1 mM of dCTP, 0.1 mM of dTTP and 0.1 mM of dGTP] were added 2.5 U of Ampli Taq DNA polymerase , 100 pmol portions of the primer represented by SEQ ID No. 24 in the Sequence Listing and the primer M13M4, 1 ng of pUscaur1R and distilled water to thereby give 100 µl of a PCR solution. This reaction mixture was then heated successively at 94°C for 1 minute, at 55°C for 1.5 minutes and at 72°C for 1.5 minutes. This cycle was repeated 30 times. Next, the PCR product was cleaved with SalI and SnaI and electrophoresed on an agarose gel. The target DNA fragment of about 1.3 kb was recovered from the gel and purified.

### (2) Construction of plasmid containing DNA coding for Aur1^{R}p (F158Y, A240C)

pUscaur1R was cleaved with SalI and SnaI and electrophoresed on an agarose gel. The target DNA fragment of 5.3 kb was recovered and purified. To this DNA fragment was ligated the DNA fragment of 1.3 kb obtained in Example 2-(1). The obtained plasmid, which had a DNA (scaur1^{R}-C) coding for Aur1^{R}p (F158Y, A240C), was named pUscaur1^{R}-C. To effect transformation by integrating it into the chromosome of sake yeast, pUscaur1^{R} -C was linearized by cleaving with StuI prior to use. As a control, pUscaurlR was also linearized with StuI.

### (3) Construction of plasmid containing DNA coding for Aur1^{R}p (A240C)

pUscaur1^{s} was cleaved with SalI and SnaI and electrophoresed on an agarose gel. The target DNA fragment of 5.3 kb was recovered and purified. This DNA fragment was ligated the DNA fragment of 1.3 kb obtained in Example 2-(1). The obtained plasmid, which had a DNA coding for Aur1^{R}p (A240C) wherein the 240th residue Ala of Aur1^{S}p had been replaced by Cys, was named pUscaur1A240C, To effect transformation by integrating it into the chromosome of sake yeast, pUscaurA240C was linearized by cleaving with StuI prior to use.

### Example 3: Transformation by using sake yeast as host

(1) About 10 µg of the linearized vector of the plasmid pAUR1 described in Example 1 was introduced into Sake yeast Kyokai k-701 by the lithium acetate method [Journal of Bacteriology, 153, 163 (1983)].
   Namely, to Sake yeast Kyokai K-701, which had been suspended in a O.1 M lithium acetate solution (about 1.3 x 10⁸cells/100 µl of 0.1 M lithium acetate), was added 10 µg of the vector which had been prepared through the linearization of scaur1^{R} by cleaving with StuI at one position. After treating at 30°C for 30 minutes and then at 42°C for 15 minutes, the cells were harvested by centrifugation and pre-incubated in 5 ml of a YPD liquid medium. After pre-incubating in a YPD liquid medium containing 0.4 µg/ml of aureobasidin A, transformants were obtained on a YPD agar medium containing 0.8 µg/ml of aureobasidin A. This transformant was named Sake yeast Kyokai K-701/pAUR1.
   This transformant was subcultured over three generations in the absence of aureobasidin A and then the sensitivity to aureobasidin was assayed. As a result, it showed eight times as much aureobasidin resistance (MIC 1.56 µg/ml) as that of the parent strain (i.e., Sake yeast Kyokai K-701), which indicated that the aureobasidin resistance was sustained. Thus it has been confirmed that the aureobasidin resistance introduced on the host chromosome is usable as a selective marker.
(2) To compare the activities of pUscaur1^{R}-C prepared in
   Example 2-(2), pUscaur1A240C prepared in Example 2-(3) and pUscaurlR prepared in Example 1-(1), 5 µg of the plasmid, which had been linearized with StuI, was introduced into Sake yeast Kyokai K-701 by the lithium acetate method. Namely, to sake yeast, which had been suspended in a 0.1 M lithium acetate solution (pH 7.5) and made competent, were added 5 µg of the plasmid, which had been linearized by cleaving with StuI at one position, and 850 µl of 40% polyethylene glycol/O.1 M lithium acetate. After treating at 30°C for 30 minutes and then maintaining at 42°C for 15 minutes, the cells were harvested, pre-incubated in 5 ml of a YPD liquid medium for 1 hour or overnight and then smeared on a YPD agar medium containing aureobasidin A at various concentration. After incubating at 30°C for 3 to 4 days, transformants having a resistance to aureobasidin A were obtained. As Table 4 shows, the transformant prepared by using the StuI-linearized pUscaur1^{R}-C could grow even in the medium containing 5 µ g/ml of aureobasidin A. These transformants sustained an aureobasidin A resistance at least 10 times higher than that of the parent strain even after being subcultured over several generations. The transformant obtained by using the linearized pUscaur1^{R}-C showed an MIC to aureobasidin A of 20 µg/ml or above. Thus it has been confirmed that the Aur1^{R}p (F158Y, A240C) is usable as an effective selective marker for sake yeast. As Table 4 shows, the StuI-linearized pUscaurlA240C exceeded the StuI-linearized pUscaurlR in the activity of imparting resistance, That is to say, the mutation at the 240th residue Ala resulted in the expression of the activity of imparting a stronger resistance.
[Table 4]

**Table 4**

| Plasmid | Pre-incubation time | No. of transformants /µg DNA | | |
|---|---|---|---|---|
| | | Aureobasidin A concn. (µg/ml) | | |
| | | 0.5 | 1.0 | 5.0 |
| StuI-linearized | 1 hr | 0 | 0 | 0 |
| pUscaur1R | overnight | 5 | 4 | 0 |
| | | | | |
| StuI-linearized | 1hr | 170 | 73 | 0 |
| pUscaur1^{R}-C | overnight | 2368 | 2024 | 64 |
| | | | | |
| StuI-linearized | 1 hr | 18 | 1 | 0 |
| pUscaur1A240C | overnight | 160 | 152 | 0 |
| | | | | |
| no plasmid | 1 hr | 0 | 0 | 0 |
| (control) | overnight | 0 | 0 | 0 |

### Example 4: Transformation by chromosome integration vector having aureobasidin resistant gene and AARE gene

### (1) Construction of plasmid containing AARE gene

0.5 µg of the plasmid pAUR1 was cleaved with SphI and electrophoresed on an agarose gel. Then the linearized plasmid pAUR1 was recovered from the gel and purified.

Next, a plasmid pYHA201 was prepared from a yeast BJ2168, which carried a plasmid pYHA201 containing the AARE gene described in Japanese Patent Laid-Open No. 254680/1991 (i.e., Saccharomyces cerevisiae BJ2168/pYHA201; FERM P-11570). 0.5 µg of this plasmid pYHA201 was digested with SphI and the DNA fragments (about 3.2 kb) were isolated and purified.

This DNA fragment of about 3.2 kb contained the AARE gene bound to the downstream of the ADHI promotor.

By using each 0.2 µg portions of both the purified SphI-cleaved DNA fragments, a ligation reaction was effected by using a DNA ligation kit (manufactured by Takara Shuzo Co., Ltd.). Subsequently, the plasmid was integrated into Escherichia coli JM109 and incubated to thereby give a transformant and a plasmid DNA. The plasmid thus obtained was named plasmid pAUR1aare, while the transformant thus obtained was named and indicated as Escherichia coli JM109/ pAUR1aare.

### (2) Expression of AARE in sake yeast

By using about 10 µg of the above-mentioned plasmid pAUR1aare, the plasmid pAUR1aare linearized was transformed into Sake yeast Kyokai K-701 in the same manner as the one described in Example 3. Then an aureobasidin resistant transformant was obtained on a YPD agar medium containing 0.4 µg/ml of aureobasidin A.

The transformant thus obtained was named and indicated as Saccharomyces cerevisiae K701/pAUR1aare.

Next, this transformant was inoculated into 5 ml of a minimal medium [0.67% of Bacto Yeast Nitrogen Base w/o Amino Acid (manufactured by Difco), 2% of glucose] and incubated at 30°C for two days and then the cells were harvested by centrifugation.

After discarding the supernatant, the cells were washed with 2 ml of water and harvested again by centrifugation.

Subsequently, the cells were suspended in 700 µl of a 0.2 M sodium phosphate buffer Solution (pH 7.2).

To this suspension were added 400 µl of glass beads (0.40 to 0.60 mm in diameter) and the cells were disrupted by vigorously stirring under ice-cooling.

After centrifuging, the supernatant was recovered and regarded as an extract.

Also, extracts of Sake yeast Kyokai K-701 and Sake yeast Kyokai K-701/pAUR1 obtained in Example 3 were prepared in the same manner.

The acylamino acid releasing enzyme activity of each of the extracts thus obtained was measured by the following method. 0.89 ml of a 0.5% dimethylformamide-0.2 M sodium phosphate buffer solution (pH 7.2) containing 0.020 mM of an amide prepared from N-acetyl-L-methionine and 7-amino-4-methylcoumarin (AMC) was preheated at 37°C for about 5 minutes. Then 100 µl of the above-mentioned extract was added thereto and the resulting mixture was incubated at 37°C for 15 minutes. After the completion of the reaction, 10 µl of 10% SDS was added to thereby cease the reaction and the intensity of fluorescence was measured with a fluorophotometer. Namely, the excitation wavelength and the measurement wavelength were set respectively to 380 nm and 440 nm. The amount of the liberated AMC was determined by preparing a standard curve with the use of AMC samples of known concentrations and comparing the obtained data therewith.

100 µl of the extract of S. cerevisiae K701/p AUR1aare had an activity of liberating about 25 pmol of AMC in 15 minutes in the above reaction system.

On the other hand, the extract of Sake yeast Kyokai K-701 having no plasmid and the extract of Sake yeast Kyokai K-701/pAUR1 obtained in Example 3 showed each no AARE activity.

Further, an analysis was effected by the southern hybridization with the use of the aureobasidin resistant gene as a probe. As a probe in the hybridization, use was made of an scaur1^{R} fragment (about 1.6 kb) which had been amplified by the PCR method with the use of the plasmid pAUR1 as a template and the primers represented by SEQ ID Nos. 16 and 17 in the Sequence Listing. 100 ng of the fragment thus obtained was labeled with [³²P] dCTP by using a BcaBEST™ labeling kit. The genomic DNAs of Sake yeast Kyokai K-701 and Saccharomyces cerevisiae K701/pAUR1aare were cleaved with various restriction enzymes (HPaI having no cleavage site on pAUR1aare, BamHI having two cleavage sites on pAuR1aare), electrophoresed on an agarose gel and transferred onto the hybridization filter.

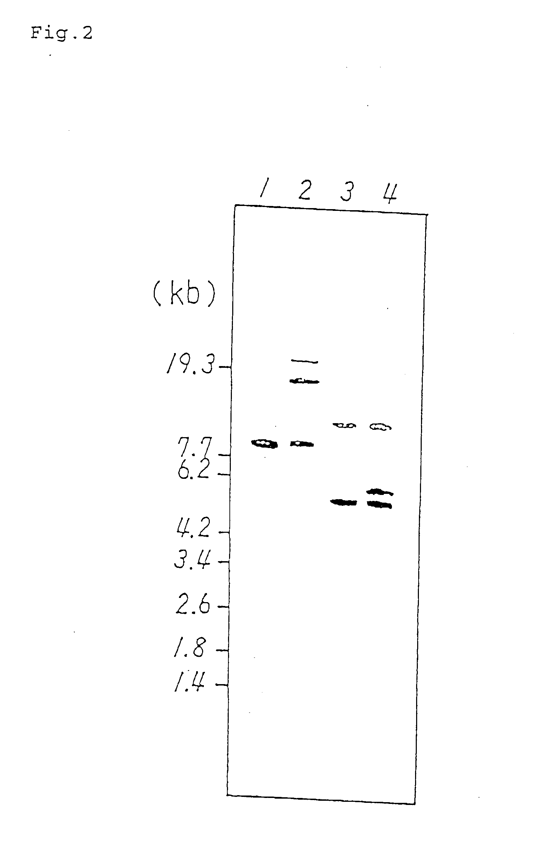

In Fig. 2, the lanes 1 and 2 show the results obtained by cleaving the genomic DNA of Sake yeast Kyokai K-701 with HpaI (lane 1) or BamHI (lane 3) and subjected to southern hybridization, while the lanes 2 and 4 show the results obtained by cleaving the genomic DNA of Saccharomyces cerevisiae K701/pAUR1aare with HpaI (lane 2) or BamHI (lane 4) and subjected to southern hybridization. Since HpaI had no cleaving site on the plasmid pAUR1aare but cleaved exclusively the genomic DNA, it was proved from the lanes 1 and 2 that the aureobasidin resistant gene had been integrated into one of a pair of chromosomes.

It was confirmed from the lanes 3 and 4 that the aureobasidin resistant gene had been homologously integrated into the aureobasidin sensitive gene on the chromosome of the sake yeast.

These result indicate that genes of the sake yeast were not disrupted by the random integration of the aureobasidin resistant gene.

As described above, by using the chromosome integration vector of the present invention, the resistance could be imparted to an aureobasidin sensitive fungi and, furthermore, a foreign gene could be expressed.

### Example 5; Construction of recombinant plasmid containing aureobasidin resistant gene

### (1) Construction of pYC vector carrying DNA coding for Aur1^{R}p (F158Y, A240C)

By employing pUscaur1^{R}-C as a template, PCR was effected with the use of primers represented by SEQ ID Nos. 25 and 26 in the Sequence Listing. The PCR product (about 2.2 kb), which contained the DNA coding for the amplified Aur1^{R}p (F158Y, A240C), was cleaved with XhoI and KpnI and blunt-ended with the use of a blunting kit (manufactured by Takara Shuzo Co., Ltd.).

A pYC vector pYEUra3 (manufactured by Clontech Laboratories, Inc.) was cleaved with EcoRI and BamHI and blunt-ended with the use of a blunting kit. Then it was ligated to the blunt-ended PCR product (about 2.2 kb) described above. The plasmid thus obtained was named pYCscaur1^{R}-C. By employing pUscaurlR as a template, PCR was effected in the same manner with the use of primers represented by SEQ ID Nos. 25 and 26 in the Sequence Listing. The PCR product (about 2.2 kb), which contained the DNA coding for the amplified Aur1^{R}p (F158Y), was cleaved with XhoI and KpnI and blunt-ended with the use of a blunting kit (manufactured by Takara Shuzo Co., Ltd.). A pYEUra3 was cleaved with EcoRI and BamHI and blunt-ended with the use of a blunting kit. Then it was ligated to the blunt-ended PCR product (about 2.2 kb) described above. The plasmid thus obtained was named pYCscaur1R.

### (2) Construction of pYE vector carrying DNA coding for Aur1^{R}p (F158Y, A240C)

By using pUscaur1^{R}-C as a template, the DNA fragment ( about 2.2 kb), which contained the DNA coding for Aur1^{R}p (F158Y, A240C), was amplified by the PCR method with the use of a primer represented by SEQ ID No.25 in the Sequence Listing and a primer M13M4. The PCR product was cleaved with BamHI and electrophoresed on an agarose gel. Then the target DNA fragment (about 1.8 kb) was recovered from the gel and purified. The plasmid pSCAR1 was cleaved with BamHI and thus a DNA fragment of 11.7 kb, from which a DNA fragment of 2.8 kb containing scaur1^{S} had been deleted, was obtained. This DNA fragment of 11.7 kb was ligated to the above-mentioned DNA fragment of 1.8 kb containing the DNA coding for Aur1^{R}p (F158Y, A240C). Then a plasmid having the fragment of 1.8 kb inserted thereinto in the desired direction was selected. This plasmid containing the DNA coding for Aur1^{R}p (F158Y, A240C) was named pWscaur1^{R}-C and employed in the transformation of a yeast for laboratory use.

### Example 6: Transformation with the use of yeast as host

### (1) Transformation of sake yeast by pYCscaur1^{R}-C

By using 5 µg of a pYC plasmid pYcscaur1^{R}-C, Sake yeast Kyokai K-701 was transformed by the lithium acetate method described in Example 1-(1). As Table 5 shows, the obtained results are similar to those obtained in the cases of the linearized plasmids. These transformants sustained each an aureobasidin A resistance at least 10 times higher than that of the parent strain even after being subcultured over several generations. Thus it has been confirmed that Aur1^{R}p (F158Y, A240C) is usable as an effective selective marker for sake yeast in the transformation by a replication vector.

[Table 5]

**Table 5**

| Plasmid | Pre-incubation time | No. of transformants/µg DNA | | | |
|---|---|---|---|---|---|
| | | Aureobasidin A concn. (µg/ml) | | | |
| | | 0.5 | 1.0 | 2.0 | 5.0 |
| pYCscaur1R | 1 hr | 0 | 0 | 0 | 0 |
| | overnight | 24 | 21 | 0 | 0 |
| | | | | | |
| pYCscaur1^{R}-C | 1 hr | 386 | 172 | 18 | 1 |
| | overnight | 4824 | 4792 | 1692 | 136 |

### (2) Transformation of laboratory yeast by pWscaur1^{R}-C

By using a monoploid yeast DKD-5D for laboratory use (a, his3, trp1, leu2-3, 112) as a host, 5µg of pWscaur1^{R}-C was transformed by the lithium acetate method described in Example 3-(1).

For comparison, a transformant was screened on a minimal medium by using an auxotrophic marker contained in the plasmid. As Table 6 shows, it has been confirmed that Aur1^{R}p (F158Y, A240C) is usable as a selective marker which is comparable to the conventional auxotrophic markers in a monoploid yeast.

[Table 6]

**Table 6**

| Plasmid | Pre-incubation time | No. of transformants/µg DNA | | | |
|---|---|---|---|---|---|
| | | Minimal Aureobasidin A concn. (µg/ml) | | | |
| | | medium | 0.5 | 1.0 | 5.0 |
| pWscaur1^{R}-C | 1 hr | 192 | 248 | 181 | 58 |
| | overnight | 2500 | 2780 | 2524 | 2044 |

### Example 7: Transformation by using C. albicans as host

5 µg of a linear plasmid, which had been prepared by cleaving pUscaur1^{R}-C with SalI capable of cleaving at one point in the pUC119 region, was transformed into C. albicans TIMM0136 by the lithium acetate method. After the completion of the transformation, the cells were incubated in a YPD medium for 1 hour or overnight and then smeared on a YPD agar medium containing aureobasidin A. As Table 7 shows, transformants having the resistance to aureobasidin were obtained by effecting the pre-incubation overnight. These transformants showed each an MIC of 10 µg/ml or above.

[Table 7]

**Table 7**

| Plasmid | Pre-incubation time | No. of transformants/µg DNA | |
|---|---|---|---|
| | | Aureobasidin A concn. (µg/ml) | |
| | | 0.25 | 1.0 |
| SalI-linearized | 1 hr | 0 | 0 |
| pUscaur1^{R}-C | overnight | 180 | 0 |

### [Effects of the Invention]

The present invention provides a chromosome integration vector with the use of a resistance to aureobasidin as a selective marker, which is useful in genetic recombination of fungi (in particular, industrial fungi), a process for producing a transformant having this vector introduced thereinto, and a transformant obtained by this process. They are effectively applicable to, for example the creation of a transformant for producing a useful protein and breeding.

The present invention also provides a protein capable of imparting a strong resistance to aureobasidin and a DNA coding for this protein. They imparted a resistance to an organism having an aureobasidin sensitivity and are useful as a selective marker for screening the organism thus acquiring the resistance. They are highly useful particularly in, for example, the genetic engineering breeding of a practically usable yeast and the analysis of genetic information of C. albicans. In particular, an aureobasidin resistant gene originating in S. cerevisiae is highly useful in the breeding of S. cerevisiae and the preparation of a transformant for producing a useful protein because S. cerevisiae is a yeast which is not only widely employed as an industrial yeast but also highly safe in genetic recombination.

### Sequence Listing

SEQ ID NO : 1
   SEQUENCE LENGTH:2385
   SEQUENCE TYPE : nucleic acid
   STRANDEDNESS : double
   TOPOLOGY: linear
   MOLECULE TYPE : Genomic DNA
   SEQUENCE DESCRIPTION : 
AAGCTTTTTT GCCTCTGCAA AAGTTCCTTT CTCGAATTGG TTTTTTGAGG AAAAGCAAGT 60
TAATAAACTA ATTATATTAT ATATAATTAG CAATTTTATA AAAAAAATAA AAAAATAGCC 120
CTGATTGCTG GCAACTGTGA GCTGAACATT GGTTAATCGG TCCATCTTTT TTTAAATATT 180
TTACATCGCT ACTTTTAAGT GCTTGACACT TGCATTTAAT AGCTACTTTC TTTCCTTCAT 240
AAAAATTCCT TTTTTTTCCT TTAGTTTTCC GGTTAATTCC TTACGAAATT TTTTTCGTAC 300
GCTTCCCTTT TTTACTCTGA TAATTCTTTG AAGCAATGTC TGCTCTTTCG ACCTTAAAAA 360
AGCGCCTTGC TGCGTGTAAC CGAGCATCCC AATACAAGTT GGAAACAAGC TTAAACCCTA 420
TGCCTACATT TCGTTTGCTA CGCAATACGA AATGGTCATG GACACATTTG CAATATGTGT 480
TTCTAGCAGG TAATTTGATT TTTGCTTGTA TTGTCATTGA ATCTCCTGGA TTCTGGGGGA 540
AATTTGGCAT TGCCTGTCTT TTGGCCATTG CGTTGACCGT TCCTTTAACA CGCCAAATTT 600
TTTTTCCTGC CATTGTTATC ATCACCTGGG CAATTTTATT TTACTCTTGT AGGTTTATTC 660
CAGAACGCTG GCGTCCACCC ATATGGGTTC GTGTTTTACC CACACTTGAA AATATTCTTT 720
ATGGCTCTAA TCTTTCTAGT CTTCTCTCGA AAACCACGCA TAGCATCCTT GATATTTTGG 780
CCTGGGTTCC ATATGGAGTC ATGCATTATT CGGCTCCTTT TATCATTTCA TTTATTCTTT 840
TCATCTTTGC ACCTCCTGGA ACTCTTCCAG TTTGGGCTCG AACTTTTGGT TATATGAATT 900
TATTTGGTGT TCTTATCCAA ATGGCTTTCC CCTGTTCTCC TCCTTGGTAT GAAAATATGT 960
ATGGTTTAGA ACCTGCCACG TATGCAGTAC GTGGCTCTCC TGGTGGATTG GCCCGTATTG 1020
ATGCTCTCTT CGGCACTAGC ATTTACACTG ATTGTTTTTC TAACTCTCCG GTTGTTTTTG 1080
GTGCCTTTCC ATCTCTTCAC GCTGGATGGG CCATGCTGGA AGCACTTTTC CTTTCGCATG 1140
TGTTTCCTCG ATACCGCTTC TGCTTTTATG GATATGTTCT ATGGCTTTGC TGGTGTACTA 1200
TGTACCTTAC CCACCACTAC TTTGTAGATT TGGTCGGCGG TATGTGTTTA GCTATTATAT 1260
 
GCTTCGTTTT TGCTCAAAAG CTACGCCTCC CACAGTTGCA AACTGGTAAA ATCCTTCGTT 1320
GGGAATACGA GTTTGTTATC CACGGTCATG GTCTTTCCGA AAAAACCAGC AACTCCTTGG 1380
CTCGTACCGG CAGCCCATAC TTACTTGGAA GGGATTCTTT TACTCAAAAC CCTAATGCAG 1440
TAGCCTTCAT GAGTGGTCTT AACAATATGG AACTTGCTAA CACCGATCAT GAATGGTCCG 1500
TGGGTTCATC ATCACCTGAG CCGTTACCTA GTCCTGCTGC TGATTTGATT GATCGTCCTG 1560
CCAGTACCAC TTCCTCCATC TTTGATGCAA GTCATCTTCC TTAAATCAAC GTGCTTTAAG 1620
AATATATTTC CAAAAGCTAC ATGATACATT GACTAGAATC GGTTTGATTC ATAGTGGTAT 1680
TGGAATGATG TTGTTCATTG TGTTTTTTAA CTGTTAATCT GACATCCATT GAGTCATTCT 1740
TTACAATTTG TAAAATTAAT TTGTATCACT AATTTTGAAG GAAGCTATTT TGGTATTAAT 1800
ACCGCTTTTG GTCTCCACTT CCTTTTCGAA ACTCTTAACA GCGATTAGGC CGGGTATCTT 1860
CCAGTGTGAT GTATAGGTAT TTGTCGTTTT TTTATCATTT CCGTTAATAA AGAACTCTTT 1920
TATCCAGCTT CTTACACTGT CAACTGTTGT GAAAGGAACA CATTTAGAAT TTCATTTTCC 1980
TTATTTGTTG TGATTTAAAT CGTTTGACAT AATTTTAAAT TTGGTTTGAA ATGTGTGTGA 2040
GAAGGCTTGT TTTATTCATT TAGTTTATTG CTTGTTTGCA CGAAAATCCA GAACGGAGCA 2100
TTAATGTAAT CCTTTTTTAT TCTGTAAAGC GTTTTTATAC AAATGTTGGT TATACGTTTC 2160
TAAAATAAGA ATATTGTTAT AATAATATAG TTTTTTCTAT CATTTGTTAC ACACACTAAA 2220
GAGACATTAA GGATAAGCAA ATGTGTTAAA ATGATAATAT ATTTTGGAAA CATTTATAAA 2280
GAAATTAAGC AGCTTTGACT AACTACATTT TTGTTTTTTT CCTAAGCAAA ACTGTATAGT 2340
TATACACGCG AGCTGTATTC ACTTCCATTG TAGTGACTTG AGCTC 2385
SEQ ID NO : 2
   SEQUENCE LENGTH : 422
   SEQUENCE TYPE : amino acid
   STRANDEDNESS : single
   TOPOLOGY : linear
   MOLECULE TYPE : peptide
   SEQUENCE DESCRIPTION : 
Met Ser Ala Leu Ser Thr Leu Lys Lys Arg Leu Ala.Ala Cys Asn
 1               5                   10                  15
 
Arg Ala Ser Gln Tyr Lys Leu Glu Thr Ser Leu Asn Pro Met Pro
20 25 30
Thr Phe Arg Leu Leu Arg Asn Thr Lys Trp Ser Trp Thr His Leu
35 40 45
Gln Tyr Val Phe Leu Ala Gly Asn Leu Ile Phe Ala Cys Ile Val
50 55 60
Ile Glu Ser Pro Gly Phe Trp Gly Lys Phe Gly Ile Ala Cys Leu
65 70 75
Leu Ala Ile Ala Leu Thr Val Pro Leu Thr Arg Gln Ile Phe Phe
80 85 90
Pro Ala Ile Val Ile Ile Thr Trp Ala Ile Leu Phe Tyr Ser Cys
95 100 105
Arg Phe Ile Pro Glu Arg Trp Arg Pro Pro Ile Trp Val Arg Val
110 115 120
Leu Pro Thr Leu Glu Asn Ile Leu Tyr Gly Ser Asn Leu Ser Ser
125 130 135
Leu Leu Ser Lys Thr Thr His Ser Ile Leu Asp Ile Leu Ala Trp
140 145 150
Val Pro Tyr Gly Val Met His Tyr Ser Ala Pro Phe Ile Ile Ser
155 160 165
Phe Ile Leu Phe Ile Phe Ala Pro Pro Gly Thr Leu Pro Val Trp
170 175 180
Ala Arg Thr Phe Gly Tyr Met Asn Leu Phe Gly Val Leu Ile Gln
185 190 195
Met Ala Phe Pro Cys Ser Pro Pro Trp Tyr Glu Asn Met Tyr Gly
200 205 210
Leu Glu Pro Ala Thr Tyr Ala Val Arg Gly Ser Pro Gly Gly Leu
215 220 225
Ala Arg Ile Asp Ala Leu Phe Gly Thr Ser Ile Tyr Thr Asp Cys
 
230 235 240
Phe Ser Asn Ser Pro Val Val Phe Gly Ala Phe Pro Ser Leu His
245 250 255
Ala Gly Trp Ala Met Leu Glu Ala Leu Phe Leu Ser His Val Phe
260 265 270
Pro Arg Tyr Arg Phe Cys Phe Tyr Gly Tyr Val Leu Trp Leu Cys
275 280 285
Trp Cys Thr Met Tyr Leu Thr His His Tyr Phe Val Asp Leu Val
290 295 300
Gly Gly Met Cys Leu Ala Ile Ile Cys Phe Val Phe Ala Gln Lys
305 310 315
Leu Arg Leu Pro Gln Leu Gln Thr Gly Lys Ile Leu Arg Trp Glu
320 325 330
Tyr Glu Phe Val Ile His Gly His Gly Leu Ser Glu Lys Thr Ser
335 340 345
Asn Ser Leu Ala Arg Thr Gly Ser Pro Tyr Leu Leu Gly Arg Asp
350 355 360
Ser Phe Thr Gln Asn Pro Asn Ala Val Ala Phe Met Ser Gly Leu
365 370 375
Asn Asn Met Glu Leu Ala Asn Thr Asp His Glu Trp Ser Val Gly
380 385 390
Ser Ser Ser Pro Glu Pro Leu Pro Ser Pro Ala Ala Asp Leu Ile
395 400 405
Asp Arg Pro Ala Ser Thr Thr Ser Ser Ile Phe Asp Ala Ser His
410 415 420
Leu Pro
SEQ ID NO :3
   SEQUENCE LENGTH : 2385
   SEQUENCE TYPE : nucleic acid
   STRANDEDNESS : double
   TOPOLOGY : linear
   MOLECULE TYPE : Genomic DNA
   SEQUENCE DESCRIPTION : 
AAGCTTTTTT GCCTCTGCAA AAGTTCCTTT CTCGAATTGG TTTTTTGAGG AAAAGCAAGT 60
TAATAAACTA ATTATATTAT ATATAATTAG CAATTTTATA AAAAAAATAA AAAAATAGCC 120
CTGATTGCTG GCAACTGTGA GCTGAACATT GGTTAATCGG TCCATCTTTT TTTAAATATT 180
TTACATCGCT ACTTTTAAGT GCTTGACACT TGCATTTAAT AGCTACTTTC TTTCCTTCAT 240
AAAAATTCCT TTTTTTTCCT TTAGTTTTCC GGTTAATTCC TTACGAAATT TTTTTCGTAC 300
GCTTCCCTTT TTTACTCTGA TAATTCTTTG AAGCAATGTC TGCTCTTTCG ACCTTAAAAA 360
AGCGCCTTGC TGCGTGTAAC CGAGCATCCC AATACAAGTT GGAAACAAGC TTAAACCCTA 420
TGCCTACATT TCGTTTGCTA CGCAATACGA AATGGTCATG GACACATTTG CAATATGTGT 480
TTCTAGCAGG TAATTTGATT TTTGCTTGTA TTGTCATTGA ATCTCCTGGA TTCTGGGGGA 540
AATTTGGCAT TGCCTGTCTT TTGGCCATTG CGTTGACCGT TCCTTTAACA CGCCAAATTT 600
TTTTTCCTGC CATTGTTATC ATCACCTGGG CAATTTTATT TTACTCTTGT AGGTTTATTC 660
CAGAACGCTG GCGTCCACCC ATATGGGTTC GTGTTTTACC CACACTTGAA AATATTCTTT 720
ATGGCTCTAA TCTTTCTAGT CTTCTCTCGA AAACCACGCA TAGCATCCTT GATATTTTGG 780
CCTGGGTTCC ATATGGAGTC ATGCATTATT CGGCTCCTTT TATCATTTCA TTTATTCTTT 840
TCATCTTTGC ACCTCCTGGA ACTCTTCCAG TTTGGGCTCG AACTTTTGGT TATATGAATT 900
TATTTGGTGT TCTTATCCAA ATGGCTTTCC CCTGTTCTCC TCCTTGGTAT GAAAATATGT 960
ATGGTTTAGA ACCTGCCACG TATGCAGTAC GTGGCTCTCC TGGTGGATTG GCCCGTATTG 1020
ATGCTCTCTT CGGCACTAGC ATTTACACTG ATGGTTTTTC TAACTCTCCG GTTGTTTTTG 1080
GTGCCTTTCC ATCTCTTCAC GCTGGATGGG CCATGCTGGA AGCACTTTTC CTTTCGCATG 1140
TGTTTCCTCG ATACCGCTTC TGCTTTTATG GATATGTTCT ATGGCTTTGC TGGTGTACTA 1200
TGTACCTTAC CCACCACTAC TTTGTAGATT TGGTCGGCGG TATGTGTTTA GCTATTATAT 1260
GCTTCGTTTT TGCTCAAAAG CTACGCCTCC CACAGTTGCA AACTGGTAAA ATCCTTCGTT 1320
GGGAATACGA GTTTGTTATC CACGGTCATG GTCTTTCCGA AAAAACCAGC AACTCCTTGG 1380
CTCGTACCGG CAGCCCATAC TTACTTGGAA GGGATTCTTT TACTCAAAAC CCTAATGCAG 1440
 
TAGCCTTCAT GAGTGGTCTT AACAATATGG AACTTGCTAA CACCGATCAT GAATGGTCCG 1500
TGGGTTCATC ATCACCTGAG CCGTTACCTA GTCCTGCTGC TGATTTGATT GATCGTCCTG 1560
CCAGTACCAC TTCCTCCATC TTTGATGCAA GTCATCTTCC TTAAATCAAC GTGCTTTAAG 1620
AATATATTTC CAAAAGCTAC ATGATACATT GACTAGAATC GGTTTGATTC ATAGTGGTAT 1680
TGGAATGATG TTGTTCATTG TGTTTTTTAA CTGTTAATCT GACATCCATT GAGTCATTCT 1740
TTACAATTTG TAAAATTAAT TTGTATCACT AATTTTGAAG GAAGCTATTT TGGTATTAAT 1800
ACCGCTTTTG GTCTCCACTT CCTTTTCGAA ACTCTTAACA GCGATTAGGC CGGGTATCTT 1860
CCAGTGTGAT GTATAGGTAT TTGTCGTTTT TTTATCATTT CCGTTAATAA AGAACTCTTT 1920
TATCCAGCTT CTTACACTGT CAACTGTTGT GAAAGGAACA CATTTAGAAT TTCATTTTCC 1980
TTATTTGTTG TGATTTAAAT CGTTTGACAT AATTTTAAAT TTGGTTTGAA ATGTGTGTGA 2040
GAAGGCTTGT TTTATTCATT TAGTTTATTG CTTGTTTGCA CGAAAATCCA GAACGGAGCA 2100
TTAATGTAAT CCTTTTTTAT TCTGTAAAGC GTTTTTATAC AAATGTTGGT TATACGTTTC 2160
TAAAATAAGA ATATTGTTAT AATAATATAG TTTTTTCTAT CATTTGTTAC ACACACTAAA 2220
GAGACATTAA GGATAAGCAA ATGTGTTAAA ATGATAATAT ATTTTGGAAA CATTTATAAA 2280
GAAATTAAGC AGCTTTGACT AACTACATTT TTGTTTTTTT CCTAAGCAAA ACTGTATAGT 2340
TATACACGCG AGCTGTATTC ACTTCCATTG TAGTGACTTG AGCTC 2385
SEQ ID NO : 4
   SEQUENCE LENGTH : 422
   SEQUENCE TYPE : amino acid
   STRANDEDNESS : single
   TOPOLOGY : linear
   MOLECULE TYPE : peptide
   SEQUENCE DESCRIPTION : 
Met Ser Ala Leu Ser Thr Leu Lys Lys Arg Leu Ala Ala Cys Asn
1               5                   10                  15
Arg Ala Ser Gln Tyr Lys Leu Glu Thr Ser Leu Asn Pro Met Pro
                20                  25                  30
Thr Phe Arg Leu Leu Arg Asn Thr Lys Trp Ser Trp Thr His Leu
 
                35                  40                  45
Gln Tyr Val Phe Leu Ala Gly Asn Leu Ile Phe Ala Cys Ile Val
                50                  55                  60
Ile Glu Ser Pro Gly Phe Trp Gly Lys Phe Gly Ile Ala Cys Leu
                65                  70                  75
Leu Ala Ile Ala Leu Thr Val Pro Leu Thr Arg Gln Ile Phe Phe
                80                  85                  90
Pro Ala Ile Val Ile Ile Thr Trp Ala Ile Leu Phe Tyr Ser Cys
                95                  100                 105
Arg Phe Ile Pro Glu Arg Trp Arg Pro Pro Ile Trp Val Arg Val
                110                 115                 120
Leu Pro Thr Leu Glu Asn Ile Leu Tyr Gly Ser Asn Leu Ser Ser
                125                 130                 135
Leu Leu Ser Lys Thr Thr His Ser Ile Leu Asp Ile Leu Ala Trp
                140                 145                 150
Val Pro Tyr Gly Val Met His Tyr Ser Ala Pro Phe Ile Ile Ser
                155                 160                 165
Phe Ile Leu Phe Ile Phe Ala Pro Pro Gly Thr Leu Pro Val Trp
                170                 175                 180
Ala Arg Thr Phe Gly Tyr Met Asn Leu Phe Gly Val Leu Ile Gln
                185                 190                 195
Met Ala Phe Pro Cys Ser Pro Pro Trp Tyr Glu Asn Met Tyr Gly
                200                 205                 210
Leu Glu Pro Ala Thr Tyr Ala Val Arg Gly Ser Pro Gly Gly Leu
                215                 220                 225
Ala Arg Ile Asp Ala Leu Phe Gly Thr Ser Ile Tyr Thr Asp Gly
                230                 235                 240
Phe Ser Asn Ser Pro Val Val Phe Gly Ala Phe Pro Ser Leu His
                245                 250                 255
 
Ala Gly Trp Ala Met Leu Glu Ala Leu Phe Leu Ser His Val Phe
                260                 265                 270
Pro Arg Tyr Arg Phe Cys Phe Tyr Gly Tyr Val Leu Trp Leu Cys
                275                 280                 285
Trp Cys Thr Met Tyr Leu Thr His His Tyr Phe Val Asp Leu Val
                290                 295                 300
Gly Gly Met Cys Leu Ala Ile Ile Cys Phe Val Phe Ala Gln Lys
                305                 310                 315
Leu Arg Leu Pro Gln Leu Gln Thr Gly Lys Ile Leu Arg Trp Glu
                320                 325                 330
Tyr Glu Phe Val Ile His Gly His Gly Leu Ser Glu Lys Thr Ser
                335                 340                 345
Asn Ser Leu Ala Arg Thr Gly Ser Pro Tyr Leu Leu Gly Arg Asp
                350                 355                 360
Ser Phe Thr Gln Asn Pro Asn Ala Val Ala Phe Met Ser Gly Leu
                365                 370                 375
Asn Asn Met Glu Leu Ala Asn Thr Asp His Glu Trp Ser Val Gly
                380                 385                 390
Ser Ser Ser Pro Glu Pro Leu Pro Ser Pro Ala Ala Asp Leu Ile
                395                 400                 405
Asp Arg Pro Ala Ser Thr Thr Ser Ser Ile Phe Asp Ala Ser His
                410                 415                 420
Leu Pro
SEQ ID NO : 5
   SEQUENCE LENGTH : 2340
   SEQUENCE TYPE : nucleic acid
   STRANDEDNESS : double
   TOPOLOGY: linear
   MOLECULE TYPE : Genomic DNA
   SEQUENCE DESCRIPTION : 
TTTCTTTCTG TCAAAGAATA ATAAAGTGCC CATCAGTGTT CATATTTGTT ACAAAGTGGT 60
TTTCTGATTT GGTACTACTG CAGAGGCGTA TTTTTTGCTT CAGTTACCAT AGCGTAAGAA 120
CACTAGCGAC TTTTGTTCGT GAACCAACAG AGTAGGATTT CTACTGCTAC ATCTCTTAGG 180
TAGTTGGTTA GTCCGATCGC TCACTTTTGG TTGTTGTTAA GTACTTCATA AGTTTATCCT 240
TTTCCTTTTT CACACTGAGC TACTTTGGGT ATAGCTTTTG GCCCAAGGAT CTTTGAATTT 300
TCTCCAAAAG TACTTTATTT TATATCCTAC AGGTTGCGGT TTTCATATTT TAAAAAGCTT 360
TTTAATCATT CCTTTGCGTA TGGCAAACCC TTTTTCGAGA TGGTTTCTAT CAGAGAGACC 420
TCCAAACTGC CATGTAGCCG ATTTAGAAAC AAGTTTAGAT CCCCATCAAA CGTTGTTGAA 480
GGTGCAAAAA TACAAACCCG CTTTAAGCGA CTGGGTGCAT TACATCTTCT TGGGATCCAT 540
CATGCTGTTT GTGTTCATTA CTAATCCCGC ACCTTGGATC TTCAAGATCC TTTTTTATTG 600
TTTCTTGGGC ACTTTATTCA TCATTCCAGC TACGTCACAG TTTTTCTTCA ATGCCTTGCC 660
CATCCTAACA TGGGTGGCGC TGTATTTCAC TTCATCGTAC TTTCCAGATG ACCGCAGGCC 720
TCCTATTACT GTCAAAGTGT TACCAGCGGT GGAAACAATT TTATACGGCG ACAATTTAAG 780
TGATATTCTT GCAACATCGA CGAATTCCTT TTTGGACATT TTAGCATGGT TACCGTACGG 840
ACTATTTCAT TATGGGGCCC CATTTGTCGT TGCTGCCATC TTATTCGTAT TTGGTCCACC 900
AACTGTTTTG CAAGGTTATG CTTTTGCATT TGGTTATATG AACCTGTTTG GTGTTATCAT 960
GCAAAATGTC TTTCCAGCCG CTCCCCCATG GTATAAAATT CTCTATGGAT TGCAATCAGC 1020
CAACTATGAT ATGCATGGCT CGCCTGGTGG ATTAGCTAGA ATTGATAAGC TACTCGGTAT 1080
TAATATGTAT ACTACAGCTT TTTCAAATTC CTCCGTCATT TTCGGTGCTT TTCCTTCACT 1140
GCATTCCGGG TGTGCTACTA TGGAAGCCCT GTTTTTCTGT TATTGTTTTC CAAAATTGAA 1200
GCCCTTGTTT ATTGCTTATG TTTGCTGGTT ATGGTGGTCA ACTATGTATC TGACACACCA 1260
TTATTTTGTA GACCTTATGG CAGGTTCTGT GCTGTCATAC GTTATTTTCC AGTACACAAA 1320
GTACACACAT TTACCAATTG TAGATACATC TCTTTTTTGC AGATGGTCAT ACACTTCAAT 1380
TGAGAAATAC GATATATCAA AGAGTGATCC ATTGGCTGCA GATTCAAACG ATATCGAAAG 1440
TGTCCCTTTG TCCAACTTGG AACTTGACTT TGATCTTAAT ATGACTGATG AACCCAGTGT 1500
AAGCCCTTCG TTATTTGATG GATCTACTTC TGTTTCTCGT TCGTCCGCCA CGTCTATAAC 1560
GTCACTAGGT GTAAAGAGGG CTTAATGAGT ATTTTATCTG CAATTACGGA TACGGTTGGT 1620
 
CTTATGTAGA TACATATAAA TATATATCTT TTTCTTTCTT TTTCTTAGTC AGGATTGTCG 1680
TTTAGCATAA TATACATGTA GTTTATTTAA TCACATACCA CTGATTATCT TTAGAATTTT 1740
ATAAATTTTT GAAATAAATG GGTGGCTTTT AATGGTGTCT ATGTTAAGTG AGGCTTTTAG 1800
AATGCTCTTC CTGCTTTGTT TATTATATGT GTATGAAAGA TATGTATGTA TTTACATGTG 1860
TTTGTAGCGT CCCCAGTCAA AACCTGTGCG CTATACCTAA ATGGATTGAT AATCTTCATT 1920
CACTAATTCT AAAATAGACT TCTTCCCCAA AGAACGGTGT AACGATGAGG CTCTATCCAG 1980
CTGCTTATCT AAATCAACTT TAACGATGGA TGATCTTATG ACACGGGGAT CTTTCTTTAA 2040
AGTTCTTAGA ATTTCAGACT GTACCGCAGC TGATGAATCA AACAGCATTA AAAAGTGATA 2100
TGCTCGAAAA TGTTTTTCCT GGTCTTTCTT CATTATTTTA GGAAGATACC TTATGCCCAT 2160
GGGTACAATG TCCCTCACCA CACCTCTGTT TTGAATAATC AGTTTCCCGA TTGTGGAAGA 2220
CAATTCTTTT GCTTCCAACT TTGGCGCATT GGAGTTGGTT ATGCGAACAA GTCCGATCAG 2280
CTCATAAAGC ATCTTAGTGA AAAGGGTGGT TTTGCGTTAT TCTTTCCTCT GTTGAAGCTT 2340
SEQ ID NO : 6
   SEQUENCE LENGTH: 401
   SEQUENCE TYPE : amino acid
   STRANDEDNESS : single
   TOPOLOGY : linear
   MOLECULE TYPE : peptide
   SEQUENCE DESCRIPTION : 
Met Ala Asn Pro Phe Ser Arg Trp Phe Leu Ser Glu Arg Pro Pro
1               5                   10                  15
Asn Cys His Val Ala Asp Leu Glu Thr Ser Leu Asp Pro His Gln
                20                  25                  30
Thr Leu Leu Lys Val Gln Lys Tyr Lys Pro Ala Leu Ser Asp Trp
                35                  40                  45
Val His Tyr Ile Phe Leu Gly Ser Ile Met Leu Phe Val Phe Ile
                50                  55                  60
Thr Asn Pro Ala Pro Trp Ile Phe Lys Ile Leu Phe Tyr Cys Phe
 
                65                  70                  75
Leu Gly Thr Leu Phe Ile Ile Pro Ala Thr Ser Gln Phe Phe Phe
                80                  85                  90
Asn Ala Leu Pro Ile Leu Thr Trp Val Ala Leu Tyr Phe Thr Ser
                95                  100                 105
Ser Tyr Phe Pro Asp Asp Arg Arg Pro Pro Ile Thr Val Lys Val
                110                 115                 120
Leu Pro Ala Val Glu Thr Ile Leu Tyr Gly Asp Asn Leu Ser Asp
                125                 130                 135
Ile Leu Ala Thr Ser Thr Asn Ser Phe Leu Asp Ile Leu Ala Trp
                140                 145                 150
Leu Pro Tyr Gly Leu Phe His Tyr Gly Ala Pro Phe Val Val Ala
                155                 160                 165
Ala Ile Leu Phe Val Phe Gly Pro Pro Thr Val Leu Gln Gly Tyr
                170                 175                 180
Ala Phe Ala Phe Gly Tyr Met Asn Leu Phe Gly Val Ile Met Gln
                185                 190                 195
Asn Val Phe Pro Ala Ala Pro Pro Trp Tyr Lys Ile Leu Tyr Gly
                200                 205                 210
Leu Gln Ser Ala Asn Tyr Asp Met His Gly Ser Pro Gly Gly Leu
                215                 220                 225
Ala Arg Ile Asp Lys Leu Leu Gly Ile Asn Met Tyr Thr Thr Ala
                230                 235                 240
Phe Ser Asn Ser Ser Val Ile Phe Gly Ala Phe Pro Ser Leu His
                245                 250                 255
Ser Gly Cys Ala Thr Met Glu Ala Leu Phe Phe Cys Tyr Cys Phe
                260                 265                 270
Pro Lys Leu Lys Pro Leu Phe Ile Ala Tyr Val Cys Trp Leu Trp
                275                 280                 285
 
Trp Ser Thr Met Tyr Leu Thr His His Tyr Phe Val Asp Leu Met
                290                 295                 300
Ala Gly Ser Val Leu Ser Tyr Val Ile Phe Gln Tyr Thr Lys Tyr
                305                 310                 315
Thr His Leu Pro Ile Val Asp Thr Ser Leu Phe Cys Arg Trp Ser
                320                 325                 330
Tyr Thr Ser Ile Glu Lys Tyr Asp Ile Ser Lys Ser Asp Pro Leu
                335                 340                 345
Ala Ala Asp Ser Asn Asp Ile Glu Ser Val Pro Leu Ser Asn Leu
                350                 355                 360
Glu Leu Asp Phe Asp Leu Asn Met Thr Asp Glu Pro Ser Val Ser
                365                 370                 375
Pro Ser Leu Phe Asp Gly Ser Thr Ser Val Ser Arg Ser Ser Ala
                380                 385                 390
Thr Ser Ile Thr Ser Leu Gly Val Lys Arg Ala
                395                 400
SEQ ID NO : 7
   SEQUENCE LENGTH : 2340
   SEQUENCE TYPE : nucleic acid
   STRANDEDNESS : double
   TOPOLOGY : linear
   MOLECULE TYPE : Genomic DNA
   SEQUENCE DESCRIPTION: 
TTTCTTTCTG TCAAAGAATA ATAAAGTGCC CATCAGTGTT CATATTTGTT ACAAAGTGGT 60
TTTCTGATTT GGTACTACTG CAGAGGCGTA TTTTTTGCTT CAGTTACCAT AGCGTAAGAA 120
CACTAGCGAC TTTTGTTCGT GAACCAACAG AGTAGGATTT CTACTGCTAC ATCTCTTAGG 180
TAGTTGGTTA GTCCGATCGC TCACTTTTGG TTGTTGTTAA GTACTTCATA AGTTTATCCT 240
TTTCCTTTTT CACACTGAGC TACTTTGGGT ATAGCTTTTG GCCCAAGGAT CTTTGAATTT 300
 
TCTCCAAAAG TACTTTATTT TATATCCTAC AGGTTGCGGT TTTCATATTT TAAAAAGCTT 360
TTTAATCATT CCTTTGCGTA TGGCAAACCC TTTTTCGAGA TGGTTTCTAT CAGAGAGACC 420
TCCAAACTGC CATGTAGCCG ATTTAGAAAC AAGTTTAGAT CCCCATCAAA CGTTGTTGAA 480
GGTGCAAAAA TACAAACCCG CTTTAAGCGA CTGGGTGCAT TACATCTTCT TGGGATCCAT 540
CATGCTGTTT GTGTTCATTA CTAATCCCGC ACCTTGGATC TTCAAGATCC TTTTTTATTG 600
TTTCTTGGGC ACTTTATTCA TCATTCCAGC TACGTCACAG TTTTTCTTCA ATGCCTTGCC 660
CATCCTAACA TGGGTGGCGC TGTATTTCAC TTCATCGTAC TTTCCAGATG ACCGCAGGCC 720
TCCTATTACT GTCAAAGTGT TACCAGCGGT GGAAACAATT TTATACGGCG ACAATTTAAG 780
TGATATTCTT GCAACATCGA CGAATTCCTT TTTGGACATT TTAGCATGGT TACCGTACGG 840
ACTATTTCAT TTTGGGGCCC CATTTGTCGT TGCTGCCATC TTATTCGTAT TTGGTCCACC 900
AACTGTTTTG CAAGGTTATG CTTTTGCATT TGGTTATATG AACCTGTTTG GTGTTATCAT 960
GCAAAATGTC TTTCCAGCCG CTCCCCCATG GTATAAAATT CTCTATGGAT TGCAATCAGC 1020
CAACTATGAT ATGCATGGCT CGCCTGGTGG ATTAGCTAGA ATTGATAAGC TACTCGGTAT 1080
TAATATGTAT ACTACAGCTT TTTCAAATTC CTCCGTCATT TTCGGTGCTT TTCCTTCACT 1140
GCATTCCGGG TGTGCTACTA TGGAAGCCCT GTTTTTCTGT TATTGTTTTC CAAAATTGAA 1200
GCCCTTGTTT ATTGCTTATG TTTGCTGGTT ATGGTGGTCA ACTATGTATC TGACACACCA 1260
TTATTTTGTA GACCTTATGG CAGGTTCTGT GCTGTCATAC GTTATTTTCC AGTACACAAA 1320
GTACACACAT TTACCAATTG TAGATACATC TCTTTTTTGC AGATGGTCAT ACACTTCAAT 1380
TGAGAAATAC GATATATCAA AGAGTGATCC ATTGGCTGCA GATTCAAACG ATATCGAAAG 1440
TGTCCCTTTG TCCAACTTGG AACTTGACTT TGATCTTAAT ATGACTGATG AACCCAGTGT 1500
AAGCCCTTCG TTATTTGATG GATCTACTTC TGTTTCTCGT TCGTCCGCCA CGTCTATAAC 1560
GTCACTAGGT GTAAAGAGGG CTTAATGAGT ATTTTATCTG CAATTACGGA TACGGTTGGT 1620
CTTATGTAGA TACATATAAA TATATATCTT TTTCTTTCTT TTTCTTAGTC AGGATTGTCG 1680
TTTAGCATAA TATACATGTA GTTTATTTAA TCACATACCA CTGATTATCT TTAGAATTTT 1740
ATAAATTTTT GAAATAAATG GGTGGCTTTT AATGGTGTCT ATGTTAAGTG AGGCTTTTAG 1800
AATGCTCTTC CTGCTTTGTT TATTATATGT GTATGAAAGA TATGTATGTA TTTACATGTG 1860
TTTGTAGCGT CCCCAGTCAA AACCTGTGCG CTATACCTAA ATGGATTGAT AATCTTCATT 1920
CACTAATTCT AAAATAGACT TCTTCCCCAA AGAACGGTGT AACGATGAGG CTCTATCCAG 1980
CTGCTTATCT AAATCAACTT TAACGATGGA TGATCTTATG ACACGGGGAT CTTTCTTTAA 2040
 
AGTTCTTAGA ATTTCAGACT GTACCGCAGC TGATGAATCA AACAGCATTA AAAAGTGATA 2100
TGCTCGAAAA TGTTTTTCCT GGTCTTTCTT CATTATTTTA GGAAGATACC TTATGCCCAT 2160
GGGTACAATG TCCCTCACCA CACCTCTGTT TTGAATAATC AGTTTCCCGA TTGTGGAAGA 2220
CAATTCTTTT GCTTCCAACT TTGGCGCATT GGAGTTGGTT ATGCGAACAA GTCCGATCAG 2280
CTCATAAAGC ATCTTAGTGA AAAGGGTGGT TTTGCGTTAT TCTTTCCTCT GTTGAAGCTT 2340
SEQ ID NO : 8
   SEQUENCE LENGTH : 401
   SEQUENCE TYPE : amino acid
   STRANDEDNESS : single
   TOPOLOGY : linear
   MOLECULE TYPE : peptide
   SEQUENCE DESCRIPTION : 
Met Ala Asn Pro Phe Ser Arg Trp Phe Leu Ser Glu Arg Pro Pro
1               5                   10                  15
Asn Cys His Val Ala Asp Leu Glu Thr Ser Leu Asp Pro His Gln
                20                  25                  30
Thr Leu Leu Lys Val Gln Lys Tyr Lys Pro Ala Leu Ser Asp Trp
                35                  40                  45
Val His Tyr Ile Phe Leu Gly Ser Ile Met Leu Phe Val Phe Ile
                50                  55                  60
Thr Asn Pro Ala Pro Trp Ile Phe Lys Ile Leu Phe Tyr Cys Phe
                65                  70                  75
Leu Gly Thr Leu Phe Ile Ile Pro Ala Thr Ser Gln Phe Phe Phe
                80                  85                  90
Asn Ala Leu Pro Ile Leu Thr Trp Val Ala Leu Tyr Phe Thr Ser
                95                  100                 105
Ser Tyr Phe Pro Asp Asp Arg Arg Pro Pro Ile Thr Val Lys Val
                110                 115                 120
 
Leu Pro Ala Val Glu Thr Ile Leu Tyr Gly Asp Asn Leu Ser Asp
                125                 130                 135
Ile Leu Ala Thr Ser Thr Asn Ser Phe Leu Asp Ile Leu Ala Trp
                140                 145                 150
Leu Pro Tyr Gly Leu Phe His Phe Gly Ala Pro Phe Val Val Ala
                155                 160                 165
Ala Ile Leu Phe Val Phe Gly Pro Pro Thr Val Leu Gln Gly Tyr
                170                 175                 180
Ala Phe Ala Phe Gly Tyr Met Asn Leu Phe Gly Val Ile Met Gln
                185                 190                 195
Asn Val Phe Pro Ala Ala Pro Pro Trp Tyr Lys Ile Leu Tyr Gly
                200                 205                 210
Leu Gln Ser Ala Asn Tyr Asp Met His Gly Ser Pro Gly Gly Leu
                215                 220                 225
Ala Arg Ile Asp Lys Leu Leu Gly Ile Asn Met Tyr Thr Thr Ala
                230                 235                 240
Phe Ser Asn Ser Ser Val Ile Phe Gly Ala Phe Pro Ser Leu His
                245                 250                 255
Ser Gly Cys Ala Thr Met Glu Ala Leu Phe Phe Cys Tyr Cys Phe
                260                 265                 270
Pro Lys Leu Lys Pro Leu Phe Ile Ala Tyr Val Cys Trp Leu Trp
                275                 280                 285
Trp Ser Thr Met Tyr Leu Thr His His Tyr Phe Val Asp Leu Met
                290                 295                 300
Ala Gly Ser Val Leu Ser Tyr Val Ile Phe Gln Tyr Thr Lys Tyr
                305                 310                 315
Thr His Leu Pro Ile Val Asp Thr Ser Leu Phe Cys Arg Trp Ser
                320                 325                 330
Tyr Thr Ser Ile Glu Lys Tyr Asp Ile Ser Lys Ser Asp Pro Leu
 
                335                 340                 345
Ala Ala Asp Ser Asn Asp Ile Glu Ser Val Pro Leu Ser Asn Leu
                350                 355                 360
Glu Leu Asp Phe Asp Leu Asn Met Thr Asp Glu Pro Ser Val Ser
                365                 370                 375
Pro Ser Leu Phe Asp Gly Ser Thr Ser Val Ser Arg Ser Ser Ala
                380                 385                 390
Thr Ser Ile Thr Ser Leu Gly Val Lys Arg Ala
                395                 400
SEQ ID NO : 9
   SEQUENCE LENGTH : 26
   SEQUENCE TYPE : nucleic acid
   STRANDEDNESS : single
   TOPOLOGY : linear
   MOLECULE TYPE : Other nucleic acid(synthetic DNA)
   SEQUENCE DESCRIPTION : TTTGGTTAYA TGAAYYTNTT YGGNGT 26
SEQ ID NO : 10
   SEQUENCE LENGTH : 29
   SEQUENCE TYPE : nucleic acid
   STRANDEDNESS : single
   TOPOLOGY: linear
   MOLECULE TYPE : Other nucleic acid(synthetic acid)
   SEQUENCE DESCRIPTION : TCTACAAART ARTGGTGNGT NARRTACAT 29
SEQ ID NO : 11
   SEQUENCE LENGTH : 2274
   SEQUENCE TYPE : nucleic acid
   STRANDEDNESS : double
   TOPOLOGY : linear
   MOLECULE TYPE : Genomic DNA
   SEQUENCE DESCRIPTION : 
TTATATATAT TATTGATTTG TTCCTGTTGT TATTTAGTTT AGAATCAGAC GACTACACCA 60
GAACCACAAT TCAACCAACA CTTATATAGA ACCTGGCTTG GAAAAAAGTA ACATTTATCA 120
TTCCTATACT TTTTTAGCAA ACATAATCCG TGTTTTACAT ATATTATTCA CCCAATATCA 180
TAACAAAAAC AAACTGAATA ATGGCGTCTT CTATTTTGCG TTCCAAAATA ATACAAAAAC 240
CGTACCAATT ATTCCACTAC TATTTTCTTC TGGAGAAGGC TCCTGGTTCT ACAGTTAGTG 300
ATTTGAATTT TGATACAAAC ATACAAACGA GTTTACGTAA ATTAAAGCAT CATCATTGGA 360
CGGTGGGAGA AATATTCCAT TATGGGTTTT TGGTTTCCAT ACTTTTTTTC GTGTTTGTGG 420
TTTTCCCAGC TTCATTTTTT ATAAAATTAC CAATAATCTT AGCATTTGCT ACTTGTTTTT 480
TAATACCCTT AACATCACAA TTTTTTCTTC CTGCCTTGCC CGTTTTCACT TGGTTGGCAT 540
TATATTTTAC GTGTGCTAAA ATACCTCAAG AATGGAAACC AGCTATCACA GTTAAAGTTT 600
TACCAGCTAT GGAAACAATT TTGTACGGCG ATAATTTATC AAATGTTTTG GCAACCATCA 660
CTACCGGAGT GTTAGATATA TTGGCATGGT TACCATATGG GATTATTCAT TTCAGTTTCC 720
CATTTGTACT TGCTGCTATT ATATTTTTAT TTGGGCCACC GACGGCATTA AGATCATTTG 780
GATTTGCCTT TGGTTATATG AACTTGCTTG GAGTCTTGAT TCAAATGGCA TTCCCAGCTG 840
CTCCTCCATG GTACAAAAAC TTGCACGGAT TAGAACCAGC TAATTATTCA ATGCACGGGT 900
CTCCTGGTGG ACTTGGAAGG ATAGATAAAT TGTTAGGTGT TGATATGTAT ACCACAGGGT 960
TTTCCAATTC ATCAATCATT TTTGGGGCAT TCCCATCGTT ACATTCAGGA TGTTGTATCA 1020
TGGAAGTGTT ATTTTTGTGT TGGTTGTTTC CACGATTCAA GTTTGTGTGG GTTACATACG 1080
CATCTTGGCT TTGGTGGAGC ACGATGTATT TGACCCATCA CTACTTTGTC GATTTGATTG 1140
GTGGAGCCAT GCTATCTTTG ACTGTTTTTG AGTTCACCAA ATATAAATAT TTGCCAAAAA 1200
ACAAAGAAGG CCTTTTCTGT CGTTGGTCAT ACACTGAAAT TGAAAAAATC GATATCCAAG 1260
AGATTGACCC TTTATCATAC AATTATATCC CTGTCAACAG CAATGATAAT GAAAGCAGAT 1320
TGTATACGAG AGTGTACCAA GAGTCTCAGG TTAGTCCCCC ACAGAGAGCT GAAACACCTG 1380
 
AAGCATTTGA GATGTCAAAT TTTTCTAGGT CTAGACAAAG CTCAAAGACT CAGGTTCCAT 1440
TGAGTAATCT TACTAACAAT GATCAAGTGT CTGGAATTAA CGAAGAGGAT GAAGAAGAAG 1500
AAGGCGATGA AATTTCATCG AGTACTCCTT CGGTGTTTGA AGACGAACCA CAGGGTAGCA 1560
CATATGCTGC ATCCTCAGCT ACATCAGTAG ATGATTTGGA TTCCAAAAGA AATTAGTAAA 1620
ATAACAGTTT CTATTAATTT CTTTATTTCC TCCTAATTAA TGATTTTATG CTCAATACCT 1680
ACACTATCTG TTTTTAATTT CCTACTTTTT TTTTATTATT GTTGAGTTCA TTTGCTGTTC 1740
ATTGAATATT TACAATTTTG CATTAATTAC CATCAATATA GAATGGGCAC AGTTTTTTTA 1800
AGTTTTTTTG TTTTTGTGTT TGTCTTTCTT TTTTTACATT AATGTGTTTG GATTGTTTTA 1860
GGTTCCTTTA TCCCTTAGCC CCCTCAGAAT ACTATTTTAT CTAATTAATT TGTTTTTATT 1920
TTCTGATATT TACCAATTGC TTTTTCTTTT GGATATTTAT AATAGCATCC CCTAATAATT 1980
AATATACAAC TGTTTCATAT ATATACGTGT ATGTCCTGTA GTGGTGGAAA CTGGAGTCAA 2040
CATTTGTATT AATGTGTACA AGAAAGCAGT GTTAATGCTA CTATTATAAT TTTTGAGGTG 2100
CAAATCAAGA GGTTGGCAGC TTTCTTATGG CTATGACCGT GAATGAAGGC TTGTAAACCA 2160
CGTAATAAAC AAAAGCCAAC AAGTTTTTTT AGAGCCTTTA ACAACATACG CAATGAGAGT 2220
GATTGCAATA CTACAAGATA TAGCCCAAAA AATTGAATGC ATTTCAACAA CAAC 2274
SEQ ID NO : 12
   SEQUENCE LENGTH : 471
   SEQUENCE TYPE : amino acid
   STRANDEDNESS : single
   TOPOLOGY : linear
   MOLECULE TYPE : peptide
   SEQUENCE DESCRIPTION : 
Met Ala Ser Ser Ile Leu Arg Ser Lys Ile Ile Gln Lys Pro Tyr
1               5                   10                  15
Gln Leu Phe His Tyr Tyr Phe Leu Ser Glu Lys Ala Pro Gly Ser
                20                  25                  30
Thr Val Ser Asp Leu Asn Phe Asp Thr Asn Ile Gln Thr Ser Leu
                35                  40                  45
 
Arg Lys Leu Lys His His His Trp Thr Val Gly Glu Ile Phe His
                50                  55                  60
Tyr Gly Phe Leu Val Ser Ile Leu Phe Phe Val Phe Val Val Phe
                65                  70                  75
Pro Ala Ser Phe Phe Ile Lys Leu Pro Ile Ile Leu Ala Phe Ala
                80                  85                  90
Thr Cys Phe Leu Ile Pro Leu Thr Ser Gln Phe Phe Leu Pro Ala
                95                  100                 105
Leu Pro Val Phe Thr Trp Leu Ala Leu Tyr Phe Thr Cys Ala Lys
                110                 115                 120
Ile Pro Gln Glu Trp Lys Pro Ala Ile Thr Val Lys Val Leu Pro
                125                 130                 135
Ala Met Glu Thr Ile Leu Tyr Gly Asp Asn Leu Ser Asn Val Leu
                140                 145                 150
Ala Thr Ile Thr Thr Gly Val Leu Asp Ile Leu Ala Trp Leu Pro
                155                 160                 165
Tyr Gly Ile Ile His Phe Ser Phe Pro Phe Val Leu Ala Ala Ile
                170                 175                 180
Ile Phe Leu Phe Gly Pro Pro Thr Ala Leu Arg Ser Phe Gly Phe
                185                 190                 195
Ala Phe Gly Tyr Met Asn Leu Leu Gly Val Leu Ile Gln Met Ala
                200                 205                 210
Phe Pro Ala Ala Pro Pro Trp Tyr Lys Asn Leu His Gly Leu Glu
                215                 220                 225
Pro Ala Asn Tyr Ser Met His Gly Ser Pro Gly Gly Leu Gly Arg
                230                 235                 240
Ile Asp Lys Leu Leu Gly Val Asp Met Tyr Thr Thr Gly Phe Ser
                245                 250                 255
Asn Ser Ser Ile Ile Phe Gly Ala Phe Pro Ser Leu His Ser Gly
 
                260                 265                 270
Cys Cys Ile Met Glu Val Leu Phe Leu Cys Trp Leu Phe Pro Arg
                275                 280                 285
Phe Lys Phe Val Trp Val Thr Tyr Ala Ser Trp Leu Trp Trp Ser
                290                 295                 300
Thr Met Tyr Leu Thr His His Tyr Phe Val Asp Leu Ile Gly Gly
                305                 310                 315
Ala Met Leu Ser Leu Thr Val Phe Glu Phe Thr Lys Tyr Lys Tyr
                320                 325                 330
Leu Pro Lys Asn Lys Glu Gly Leu Phe Cys Arg Trp Ser Tyr Thr
                335                 340                 345
Glu Ile Glu Lys Ile Asp Ile Gln Glu Ile Asp Pro Leu Ser Tyr
                350                 355                 360
Asn Tyr Ile Pro Val Asn Ser Asn Asp Asn Glu Ser Arg Leu Tyr
                365                 370                 375
Thr Arg Val Tyr Gln Glu Ser Gln Val Ser Pro Pro Gln Arg Ala
                380                 385                 390
Glu Thr Pro Glu Ala Phe Glu Met Ser Asn Phe Ser Arg Ser Arg
                395                 400                 405
Gln Ser Ser Lys Thr Gln Val Pro Leu Ser Asn Leu Thr Asn Asn
                410                 415                 420
Asp Gln Val Ser Gly Ile Asn Glu Glu Asp Glu Glu Glu Glu Gly
                425                 430                 435
Asp Glu Ile Ser Ser Ser Thr Pro Ser Val Phe Glu Asp Glu Pro
                440                 445                 450
Gln Gly Ser Thr Tyr Ala Ala Ser Ser Ala Thr Ser Val Asp Asp
                455                 460                 465
Leu Asp Ser Lys Arg Asn
                470
SEQ ID NO : 13
   SEQUENCE LENGTH : 22
   SEQUENCE TYPE : nucleic acid
   STRANDEDNESS : single
   TOPOLOGY : linear
   MOLECULE TYPE : Other nucleic acid(synthetic DNA)
   SEQUENCE DESCRIPTION : GACTATTTCA TTATGGGGCC CC 22
SEQ ID NO : 14
   SEQUENCE LENGTH : 30
   SEQUENCE TYPE : nucleic acid
   STRANDEDNESS : single
   TOPOLOGY: linear
   MOLECULE TYPE : Other nucleic acid(synthetic DNA)
   SEQUENCE DESCRIPTION : GTTAACTCGA GAAAGTGCCC ATCAGTGTTC 30
SEQ ID NO: 15
   SEQUENCE LENGTH : 29
   SEQUENCE TYPE : nucleic acid
   STRANDEDNESS : single
   TOPOLOGY : linear
   MOLECULE TYPE : Other nucleic acid(synthetic DNA) SEQUENCE DESCRIPTION : GTTAACGGTA CCTCATCGTT ACACCGTTC 29
SEQ ID NO : 16
   SEQUENCE LENGTH : 19
   SEQUENCE TYPE : nucleic acid
   STRANDEDNESS : single
   TOPOLOGY : linear
   MOLECULE TYPE : Other nucleic acid(synthetic DNA)
   SEQUENCE DESCRIPTION : GCTAAACGAC AATCCTGAC 19
SEQ ID NO : 17
   SEQUENCE LENGTH : 21
   SEQUENCE TYPE : nucleic acid
   STRANDEDNESS : single
   TOPOLOGY : linear
   MOLECULE TYPE : Other nucleic acid(synthetic DNA)
   SEQUENCE DESCRIPTION : CGTTGGCCGA TTCATTAATG C 21
SEQ ID NO : 18
   SEQUENCE LENGTH : 401
   SEQUENCE TYPE : amino acid
   STRANDEDNESS: single
   TOPOLOGY : linear
   MOLECULE TYPE : peptide
   SEQUENCE DESCRIPTION : 
Met Ala Asn Pro Phe Ser Arg Trp Phe Leu Ser Glu Arg Pro Pro
1               5                   10                  15
Asn Cys His Val Ala Asp Leu Glu Thr Ser Leu Asp Pro His Gln
                20                  25                  30
Thr Leu Leu Lys Val Gln Lys Tyr Lys Pro Ala Leu Ser Asp Trp
                35                  40                  45
 
Val His Tyr Ile Phe Leu Gly Ser Ile Met Leu Phe Val Phe Ile
                50                  55                  60
Thr Asn Pro Ala Pro Trp Ile Phe Lys Ile Leu Phe Tyr Cys Phe
                65                  70                  75
Leu Gly Thr Leu Phe Ile Ile Pro Ala Thr Ser Gln Phe Phe Phe
                80                  85                  90
Asn Ala Leu Pro Ile Leu Thr Trp Val Ala Leu Tyr Phe Thr Ser
                95                  100                 105
Ser Tyr Phe Pro Asp Asp Arg Arg Pro Pro Ile Thr Val Lys Val
                110                 115                 120
Leu Pro Ala Val Glu Thr Ile Leu Tyr Gly Asp Asn Leu Ser Asp
                125                 130                 135
Ile Leu Ala Thr Ser Thr Asn Ser Phe Leu Asp Ile Leu Ala Trp
                140                 145                 150
Leu Pro Tyr Gly Leu Phe His Phe Gly Ala Pro Phe Val Val Ala
                155                 160                 165
Ala Ile Leu Phe Val Phe Gly Pro Pro Thr Val Leu Gln Gly Tyr
                170                 175                 180
Ala Phe Ala Phe Gly Tyr Met Asn Leu Phe Gly Val Ile Met Gln
                185                 190                 195
Asn Val Phe Pro Ala Ala Pro Pro Trp Tyr Lys Ile Leu Tyr Gly
                200                 205                 210
Leu Gln Ser Ala Asn Tyr Asp Met His Gly Ser Pro Gly Gly Leu
                215                 220                 225
Ala Arg Ile Asp Lys Leu Leu Gly Ile Asn Met Tyr Thr Thr Cys
                230                 235                 240
Phe Ser Asn Ser Ser Val Ile Phe Gly Ala Phe Pro Ser Leu His
                245                 250                 255
Ser Gly Cys Ala Thr Met Glu Ala Leu Phe Phe Cys Tyr Cys Phe
 
                260                 265                 270
Pro Lys Leu Lys Pro Leu Phe Ile Ala Tyr Val Cys Trp Leu Trp
                275                 280                 285
Trp Ser Thr Met Tyr Leu Thr His His Tyr Phe Val Asp Leu Met
                290                 295                 300
Ala Gly Ser Val Leu Ser Tyr Val Ile Phe Gln Tyr Thr Lys Tyr
                305                 310                 315
Thr His Leu Pro Ile Val Asp Thr Ser Leu Phe Cys Arg Trp Ser
                320                 325                 330
Tyr Thr Ser Ile Glu Lys Tyr Asp Ile Ser Lys Ser Asp Pro Leu
                335                 340                 345
Ala Ala Asp Ser Asn Asp Ile Glu Ser Val Pro Leu Ser Asn Leu
                350                 355                 360
Glu Leu Asp Phe Asp Leu Asn Met Thr Asp Glu Pro Ser Val Ser
                365                 370                 375
Pro Ser Leu Phe Asp Gly Ser Thr Ser Val Ser Arg Ser Ser Ala
                380                 385                 390
Thr Ser Ile Thr Ser Leu Gly Val Lys Arg Ala
                395                 400
SEQ ID NO : 19
   SEQUENCE LENGTH : 401
   SEQUENCE TYPE : amino acid
   STRANDEDNESS : single
   TOPOLOGY : linear
   MOLECULE TYPE : peptide
   SEQUENCE DESCRIPTION : 
Met Ala Asn Pro Phe Ser Arg Trp Phe Leu Ser Glu Arg Pro Pro
1               5                   10                  15
Asn Cys His Val Ala Asp Leu Glu Thr Ser Leu Asp Pro His Gln
 
                20                  25                  30
Thr Leu Leu Lys Val Gln Lys Tyr Lys Pro Ala Leu Ser Asp Trp
                35                  40                  45
Val His Tyr Ile Phe Leu Gly Ser Ile Met Leu Phe Val Phe Ile
                50                  55                  60
Thr Asn Pro Ala Pro Trp Ile Phe Lys Ile Leu Phe Tyr Cys Phe
                65                  70                  75
Leu Gly Thr Leu Phe Ile Ile Pro Ala Thr Ser Gln Phe Phe Phe
                80                  85                  90
Asn Ala Leu Pro Ile Leu Thr Trp Val Ala Leu Tyr Phe Thr Ser
                95                  100                 105
Ser Tyr Phe Pro Asp Asp Arg Arg Pro Pro Ile Thr Val Lys Val
                110                 115                 120
Leu Pro Ala Val Glu Thr Ile Leu Tyr Gly Asp Asn Leu Ser Asp
                125                 130                 135
Ile Leu Ala Thr Ser Thr Asn Ser Phe Leu Asp Ile Leu Ala Trp
                140                 145                 150
Leu Pro Tyr Gly Leu Phe His Tyr Gly Ala Pro Phe Val Val Ala
                155                 160                 165
Ala Ile Leu Phe Val Phe Gly Pro Pro Thr Val Leu Gln Gly Tyr
                170                 175                 180
Ala Phe Ala Phe Gly Tyr Met Asn Leu Phe Gly Val Ile Met Gln
                185                 190                 195
Asn Val Phe Pro Ala Ala Pro Pro Trp Tyr Lys Ile Leu Tyr Gly
                200                 205                 210
Leu Gln Ser Ala Asn Tyr Asp Met His Gly Ser Pro Gly Gly Leu
                215                 220                 225
Ala Arg Ile Asp Lys Leu Leu Gly Ile Asn Met Tyr Thr Thr Cys
                230                 235                 240
 
Phe Ser Asn Ser Ser Val Ile Phe Gly Ala Phe Pro Ser Leu His
                245                 250                 255
Ser Gly Cys Ala Thr Met Glu Ala Leu Phe Phe Cys Tyr Cys Phe
                260                 265                 270
Pro Lys Leu Lys Pro Leu Phe Ile Ala Tyr Val Cys Trp Leu Trp
                275                 280                 285
Trp Ser Thr Met Tyr Leu Thr His His Tyr Phe Val Asp Leu Met
                290                 295                 300
Ala Gly Ser Val Leu Ser Tyr Val Ile Phe Gln Tyr Thr Lys Tyr
                305                 310                 315
Thr His Leu Pro Ile Val Asp Thr Ser Leu Phe Cys Arg Trp Ser
                320                 325                 330
Tyr Thr Ser Ile Glu Lys Tyr Asp Ile Ser Lys Ser Asp Pro Leu
                335                 340                 345
Ala Ala Asp Ser Asn Asp Ile Glu Ser Val Pro Leu Ser Asn Leu
                350                 355                 360
Glu Leu Asp Phe Asp Leu Asn Met Thr Asp Glu Pro Ser Val Ser
                365                 370                 375
Pro Ser Leu Phe Asp Gly Ser Thr Ser Val Ser Arg Ser Ser Ala
                380                 385                 390
Thr Ser Ile Thr Ser Leu Gly Val Lys Arg Ala
                395                 400
SEQ ID NO : 20
   SEQUENCE LENGTH : 1206
   SEQUENCE TYPE : nucleic acid
   STRANDEDNESS : double
   TOPOLOGY : linear
   MOLECULE TYPE : Genomic DNA
   SEQUENCE DESCRIPTION : 
ATGGCAAACC CTTTTTCGAG ATGGTTTCTA TCAGAGAGAC CTCCAAACTG CCATGTAGCC 60
GATTTAGAAA CAAGTTTAGA TCCCCATCAA ACGTTGTTGA AGGTGCAAAA ATACAAACCC 120
GCTTTAAGCG ACTGGGTGCA TTACATCTTC TTGGGATCCA TCATGCTGTT TGTGTTCATT 180
ACTAATCCCG CACCTTGGAT CTTCAAGATC CTTTTTTATT GTTTCTTGGG CACTTTATTC 240
ATCATTCCAG CTACGTCACA GTTTTTCTTC AATGCCTTGC CCATCCTAAC ATGGGTGGCG 300
CTGTATTTCA CTTCATCGTA CTTTCCAGAT GACCGCAGGC CTCCTATTAC TGTCAAAGTG 360
TTACCAGCGG TGGAAACAAT TTTATACGGC GACAATTTAA GTGATATTCT TGCAACATCG 420
ACGAATTCCT TTTTGGACAT TTTAGCATGG TTACCGTACG GACTATTTCA TTTTGGGGCC 480
CCATTTGTCG TTGCTGCCAT CTTATTCGTA TTTGGTCCAC CAACTGTTTT GCAAGGTTAT 540
GCTTTTGCAT TTGGTTATAT GAACCTGTTT GGTGTTATCA TGCAAAATGT CTTTCCAGCC 600
GCTCCCCCAT GGTATAAAAT TCTCTATGGA TTGCAATCAG CCAACTATGA TATGCATGGC 660
TCGCCTGGTG GATTAGCTAG AATTGATAAG CTACTCGGTA TTAATATGTA TACTACATGT 720
TTTTCAAATT CCTCCGTCAT TTTCGGTGCT TTTCCTTCAC TGCATTCCGG GTGTGCTACT 780
ATGGAAGCCC TGTTTTTCTG TTATTGTTTT CCAAAATTGA AGCCCTTGTT TATTGCTTAT 840
GTTTGCTGGT TATGGTGGTC AACTATGTAT CTGACACACC ATTATTTTGT AGACCTTATG 900
GCAGGTTCTG TGCTGTCATA CGTTATTTTC CAGTACACAA AGTACACACA TTTACCAATT 960
GTAGATACAT CTCTTTTTTG CAGATGGTCA TACACTTCAA TTGAGAAATA CGATATATCA 1020
AAGAGTGATC CATTGGCTGC AGATTCAAAC GATATCGAAA GTGTCCCTTT GTCCAACTTG 1080
GAACTTGACT TTGATCTTAA TATGACTGAT GAACCCAGTG TAAGCCCTTC GTTATTTGAT 1140
GGATCTACTT CTGTTTCTCG TTCGTCCGCC ACGTCTATAA CGTCACTAGG TGTAAAGAGG 1200
GCTTAA 1206
SEQ ID NO: 21
   SEQUENCE LENGTH : 1206
   SEQUENCE TYPE : nucleic acid
   STRANDEDNESS : double
   TOPOLOGY : linear
   MOLECULE TYPE : Genomic DNA
   SEQUENCE DESCRIPTION : 
ATGGCAAACC CTTTTTCGAG ATGGTTTCTA TCAGAGAGAC CTCCAAACTG CCATGTAGCC 60
GATTTAGAAA CAAGTTTAGA TCCCCATCAA ACGTTGTTGA AGGTGCAAAA ATACAAACCC 120
GCTTTAAGCG ACTGGGTGCA TTACATCTTC TTGGGATCCA TCATGCTGTT TGTGTTCATT 180
ACTAATCCCG CACCTTGGAT CTTCAAGATC CTTTTTTATT GTTTCTTGGG CACTTTATTC 240
ATCATTCCAG CTACGTCACA GTTTTTCTTC AATGCCTTGC CCATCCTAAC ATGGGTGGCG 300
CTGTATTTCA CTTCATCGTA CTTTCCAGAT GACCGCAGGC CTCCTATTAC TGTCAAAGTG 360
TTACCAGCGG TGGAAACAAT TTTATACGGC GACAATTTAA GTGATATTCT TGCAACATCG 420
ACGAATTCCT TTTTGGACAT TTTAGCATGG TTACCGTACG GACTATTTCA TTATGGGGCC 480
CCATTTGTCG TTGCTGCCAT CTTATTCGTA TTTGGTCCAC CAACTGTTTT GCAAGGTTAT 540
GCTTTTGCAT TTGGTTATAT GAACCTGTTT GGTGTTATCA TGCAAAATGT CTTTCCAGCC 600
GCTCCCCCAT GGTATAAAAT TCTCTATGGA TTGCAATCAG CCAACTATGA TATGCATGGC 660
TCGCCTGGTG GATTAGCTAG AATTGATAAG CTACTCGGTA TTAATATGTA TACTACATGT 720
TTTTCAAATT CCTCCGTCAT TTTCGGTGCT TTTCCTTCAC TGCATTCCGG GTGTGCTACT 780
ATGGAAGCCC TGTTTTTCTG TTATTGTTTT CCAAAATTGA AGCCCTTGTT TATTGCTTAT 840
GTTTGCTGGT TATGGTGGTC AACTATGTAT CTGACACACC ATTATTTTGT AGACCTTATG 900
GCAGGTTCTG TGCTGTCATA CGTTATTTTC CAGTACACAA AGTACACACA TTTACCAATT 960
GTAGATACAT CTCTTTTTTG CAGATGGTCA TACACTTCAA TTGAGAAATA CGATATATCA 1020
AAGAGTGATC CATTGGCTGC AGATTCAAAC GATATCGAAA GTGTCCCTTT GTCCAACTTG 1080
GAACTTGACT TTGATCTTAA TATGACTGAT GAACCCAGTG TAAGCCCTTC GTTATTTGAT 1140
GGATCTACTT CTGTTTCTCG TTCGTCCGCC ACGTCTATAA CGTCACTAGG TGTAAAGAGG 1200
GCTTAA 1206
SEQ ID NO : 22
   SEQUENCE LENGTH : 1206
   SEQUENCE TYPE : nucleic acid
   STRANDEDNESS : double
   TOPOLOGY : linear
   MOLECULE TYPE : Genomic DNA
   SEQUENCE DESCRIPTION : 
ATGGCAAACC CTTTTTCGAG ATGGTTTCTA TCAGAGAGAC CTCCAAACTG CCATGTAGCC 60
GATTTAGAAA CAAGTTTAGA TCCCCATCAA ACGTTGTTGA AGGTGCAAAA ATACAAACCC 120
GCTTTAAGCG ACTGGGTGCA TTACATCTTC TTGGGATCCA TCATGCTGTT TGTGTTCATT 180
ACTAATCCCG CACCTTGGAT CTTCAAGATC CTTTTTTATT GTTTCTTGGG CACTTTATTC 240
ATCATTCCAG CTACGTCACA GTTTTTCTTC AATGCCTTGC CCATCCTAAC ATGGGTGGCG 300
CTGTATTTCA CTTCATCGTA CTTTCCAGAT GACCGCAGGC CTCCTATTAC TGTCAAAGTG 360
TTACCAGCGG TGGAAACAAT TTTATACGGC GACAATTTAA GTGATATTCT TGCAACATCG 420
ACGAATTCCT TTTTGGACAT TTTAGCATGG TTACCGTACG GACTATTTCA TTATGGGGCC 480
CCATTTGTCG TTGCTGCCAT CTTATTCGTA TTTGGTCCAC CAACTGTTTT GCAAGGTTAT 540
GCTTTTGCAT TTGGTTATAT GAACCTGTTT GGTGTTATCA TGCAAAATGT CTTTCCAGCC 600
GCTCCCCCAT GGTATAAAAT TCTCTATGGA TTGCAATCAG CCAACTATGA TATGCATGGC 660
TCGCCTGGTG GATTAGCTAG AATTGATAAG CTACTCGGTA TTAATATGTA TACTACAGCT 720
TTTTCAAATT CCTCCGTCAT TTTCGGTGCT TTTCCTTCAC TGCATTCCGG GTGTGCTACT 780
ATGGAAGCCC TGTTTTTCTG TTATTGTTTT CCAAAATTGA AGCCCTTGTT TATTGCTTAT 840
GTTTGCTGGT TATGGTGGTC AACTATGTAT CTGACACACC ATTATTTTGT AGACCTTATG 900
GCAGGTTCTG TGCTGTCATA CGTTATTTTC CAGTACACAA AGTACACACA TTTACCAATT 960
GTAGATACAT CTCTTTTTTG CAGATGGTCA TACACTTCAA TTGAGAAATA CGATATATCA 1020
AAGAGTGATC CATTGGCTGC AGATTCAAAC GATATCGAAA GTGTCCCTTT GTCCAACTTG 1080
GAACTTGACT TTGATCTTAA TATGACTGAT GAACCCAGTG TAAGCCCTTC GTTATTTGAT 1140
GGATCTACTT CTGTTTCTCG TTCGTCCGCC ACGTCTATAA CGTCACTAGG TGTAAAGAGG 1200
GCTTAA 1206
SEQ ID NO : 23
   SEQUENCE LENGTH : 1206
   SEQUENCE TYPE : nucleic acid
   STRANDEDNESS : double
   TOPOLOGY: linear
   MOLECULE TYPE : Genomic DNA
   SEQUENCE DESCRIPTION : 
ATGGCAAACC CTTTTTCGAG ATGGTTTCTA TCAGAGAGAC CTCCAAACTG CCATGTAGCC 60
GATTTAGAAA CAAGTTTAGA TCCCCATCAA ACGTTGTTGA AGGTGCAAAA ATACAAACCC 120
GCTTTAAGCG ACTGGGTGCA TTACATCTTC TTGGGATCCA TCATGCTGTT TGTGTTCATT 180
ACTAATCCCG CACCTTGGAT CTTCAAGATC CTTTTTTATT GTTTCTTGGG CACTTTATTC 240
ATCATTCCAG CTACGTCACA GTTTTTCTTC AATGCCTTGC CCATCCTAAC ATGGGTGGCG 300
CTGTATTTCA CTTCATCGTA CTTTCCAGAT GACCGCAGGC CTCCTATTAC TGTCAAAGTG 360
TTACCAGCGG TGGAAACAAT TTTATACGGC GACAATTTAA GTGATATTCT TGCAACATCG 420
ACGAATTCCT TTTTGGACAT TTTAGCATGG TTACCGTACG GACTATTTCA TTTTGGGGCC 480
CCATTTGTCG TTGCTGCCAT CTTATTCGTA TTTGGTCCAC CAACTGTTTT GCAAGGTTAT 540
GCTTTTGCAT TTGGTTATAT GAACCTGTTT GGTGTTATCA TGCAAAATGT CTTTCCAGCC 600
GCTCCCCCAT GGTATAAAAT TCTCTATGGA TTGCAATCAG CCAACTATGA TATGCATGGC 660
TCGCCTGGTG GATTAGCTAG AATTGATAAG CTACTCGGTA TTAATATGTA TACTACAGCT 720
TTTTCAAATT CCTCCGTCAT TTTCGGTGCT TTTCCTTCAC TGCATTCCGG GTGTGCTACT 780
ATGGAAGCCC TGTTTTTCTG TTATTGTTTT CCAAAATTGA AGCCCTTGTT TATTGCTTAT 840
GTTTGCTGGT TATGGTGGTC AACTATGTAT CTGACACACC ATTATTTTGT AGACCTTATG 900
GCAGGTTCTG TGCTGTCATA CGTTATTTTC CAGTACACAA AGTACACACA TTTACCAATT 960
GTAGATACAT CTCTTTTTTG CAGATGGTCA TACACTTCAA TTGAGAAATA CGATATATCA 1020
AAGAGTGATC CATTGGCTGC AGATTCAAAC GATATCGAAA GTGTCCCTTT GTCCAACTTG 1080
GAACTTGACT TTGATCTTAA TATGACTGAT GAACCCAGTG TAAGCCCTTC GTTATTTGAT 1140
GGATCTACTT CTGTTTCTCG TTCGTCCGCC ACGTCTATAA CGTCACTAGG TGTAAAGAGG 1200
GCTTAA 1206
SEQ ID NO : 24
   SEQUENCE LENGTH : 30
   SEQUENCE TYPE : nucleic acid
   STRANDEDNESS : single
   TOPOLOGY : linear
   MOLECULE TYPE : Other nucleic acid(synthetic DNA)
   SEQUENCE DESCRIPTION : AATATGTATA CTACATGTTT TTCAAATTCC 30
SEQ ID NO : 25
   SEQUENCE LENGTH : 30
   SEQUENCE TYPE : nucleic acid
   STRANDEDNESS : single
   TOPOLOGY : linear
   MOLECULE TYPE : Other nucleic acid(synthetic DNA)
   SEQUENCE DESCRIPTION : GTTAACTCGA GAAAGTGCCC ATCAGTGTTC 30
SEQ ID NO : 26
   SEQUENCE LENGTH : 29
   SEQUENCE TYPE : nucleic acid
   STRANDEDNESS : single
   TOPOLOGY : linear
   MOLECULE TYPE : Other nucleic acid(synthetic DNA)
   SEQUENCE DESCRIPTION :

GTTAACGGTA CCAGAGGAAA GAATAACGC 29

## Claims

1. A chromosome integration vector for a host fungus characterized in that it contains an aureobasidin resistant gene.

2. A vector as claimed in Claim 1 which contains a foreign gene.

3. A process for producing an aureobasidin resistant transformant comprising the steps of:
(1) obtaining a replication vector which contains an aureobasidin resistant gene,
(2) cleaving the aureobasidin resistant gene in the replication vector obtained in the above step at one site to give a chromosome integration vector for a host fungus;
(3) integrating the chromosome integration vector for a host fungus obtained in the above step into the chromosome of the host fungus; and
(4) selecting a host which has been transformed into an aureobasidin resistant one in the presence of aureobasidin.

4. A process for producing a transformant as claimed in Claim 3 characterized in that the replication vector described in Claim 3 is one containing a foreign gene.

5. A protein capable of imparting aureobasidin resistance, wherein at least the 240th amino acid residue Ala in the protein capable of imparting aureobasidin sensitivity represented by SEQ ID No. 8 in the Sequence Listing has been replaced by another amino acid residue, or another protein capable of imparting aureobasidin resistance which has an amino acid sequence obtained by subjecting the above-mentioned protein to at least one modification selected from replacement, insertion and deletion of amino acid residue(s) and shows a biological activity comparable to that of said protein.

6. A protein capable of imparting aureobasidin resistance as claimed in Claim 5 in which the 240th amino acid residue Ala has been replaced by Cys.

7. A protein capable of imparting aureobasidin resistance as claimed in Claim 6 represented by SEQ ID No. 18 or 19 in the Sequence Listing.

8. A DNA coding for a protein capable of imparting aureobasidin resistance as claimed in Claim 5, 6 or 7.

9. A DNA as claimed in Claim 8 represented by SEQ ID No. 20 or 21 in the Sequence Listing.

10. A chromosome integration vector for a host fungus as claimed in Claim 1 or 2 in which the said aureobasidin resistant gene is a DNA as claimed in Claim 8 or 9.

11. A process for producing a transformant as claimed in Claim 3 or 4 wherein said aureobasidin resistant gene is a DNA as claimed in Claim 8 or 9.

## Patentansprüche

1. Chromosomaler Integrationsvektor für einen Wirtspilz, dadurch gekennzeichnet, daß er ein Aureobasidin-Resistenzgen enthält.

2. Vektor nach Anspruch 1, der ein Fremdgen enthält.

3. Verfahren zur Herstellung eines Aureobasidin-resistenten Transformanten, umfassend die Schritte:
(1) Erhalten eines Replikationsvektors, der ein Aureobasidin-Resistenzgen enthält,
(2) Spalten des Aureobasidin-Resistenzgens im nach vorstehendem Schritt erhaltenen Replikationsvektors an einer Stelle zum Erhalt eines chromosomalen Integrationsvektors für einen Wirtspilz,
(3) Integrieren des im vorstehenden Schritt erhaltenen chromosomalen Integrationsvektor für einen Wirtspilz in das Chromosom des Wirtspilz und
(4) Selektieren eines Wirts, der zu einem Aureobasidin-Resistenten transformiert worden ist, in Gegenwart von Aureobasidin.

4. Verfahren zum Herstellen eines Transformanten nach Anspruch 3, dadurch gekennzeichnet, daß der in Anspruch 3 beschriebene Replikationsvektor ein Fremdgen-enthaltender ist.

5. Protein, befähigt zur Verleihung einer Aureobasidin-Resistenz, wobei mindestens der 240te Aminosäurerest Ala in dem Protein, befähigt zur Verleihung einer Aureobasidin-Sensitivität, dargestellt durch SEQ ID Nr. 8 im Sequenzprotokoll, durch einen anderen Aminosäurerest ersetzt worden ist, oder ein anderes Protein, befähigt zur Verleihung einer Aureobasidin-Resistenz, welches eine Aminosäuresequenz aufweist, die durch Unterwerfen des vorstehend aufgeführten Proteins zu mindestens einer Modifikation, ausgewählt aus Ersetzen, Insertion und Deletion von (einem) Aminosäurerest(en) erhalten ist und eine biologische Aktivität zeigt, welche zu der des Proteins vergleichbar ist.

6. Protein, befähigt zur Verleihung einer Aureobasidin-Resistenz, nach Anspruch 5, in welchem der 240te Aminosäurerest Ala durch Cys ersetzt worden ist.

7. Protein, befähigt zur Verleihung einer Aureobasidin-Resistenz, nach Anspruch 6, dargestellt durch SEQ ID Nr. 18 oder 19 im Sequenzprotokoll.

8. DNA, kodierend für ein Protein, befähigt zur Verleihung einer Aureobasidin-Resistenz nach Anspruch 5, 6 oder 7.

9. DNA nach Anspruch 8, dargestellt durch SEQ ID Nr. 20 oder 21 im Sequenzprotokoll.

10. Chromosomaler Integrationsvektor für einen Wirtspilz nach Anspruch 1 oder 2, in welchem das Aureobasidin-Resistenzgen eine DNA nach Anspruch 8 oder 9 ist.

11. Verfahren zur Herstellung eines Transformanten nach Anspruch 3 oder 4, wobei das Aureobasidin-Resistenzgen eine DNA nach Anspruch 8 oder 9 ist.

## Revendications

1. Vecteur d'intégration chromosomique pour un champignon hôte, caractérisé en ce qu'il contient un gène de résistance à l'auréobasidine.

2. Vecteur suivant la revendication 1, qui contient un gène étranger.

3. Procédé pour la production d'un transformant résistant à l'auréobasidine, comprenant les étapes consistant :
(1) à obtenir un vecteur de réplication qui contient un gène de résistance à l'auréobasidine,
(2) à cliver le gène de résistance à l'auréobasidine dans le vecteur de réplication obtenu dans l'étape ci-dessus au niveau d'un site pour obtenir un vecteur d'intégration chromosomique pour un champignon hôte ;
(3) à intégrer le vecteur d'intégration chromosomique pour un champignon hôte obtenu dans l'étape ci-dessus dans le chromosome du champignon hôte ; et
(4) à sélectionner un hôte qui a été transformé en un hôte résistant à l'auréobasidine en présence d'auréobasidine.

4. Procédé pour la production d'un transformant suivant la revendication 3, caractérisé en ce que le vecteur de réplication décrit dans la revendication 3 est un vecteur contenant un gène étranger.

5. Protéine capable de conférer une résistance à l'auréobasidine, dans laquelle au moins le 240ème résidu d'aminoacide Ala dans la protéine, capable de conférer une sensibilité à l'auréobasidine représentée par la SEQ ID N° 8 dans la liste des séquences, a été remplacé par un autre résidu d'aminoacide, ou une autre protéine capable de conférer une résistance à l'auréobasidine qui a une séquence d'aminoacides obtenue en soumettant la protéine précitée à au moins une modification choisie entre un remplacement, une insertion et une délétion d'un ou plusieurs résidus d'aminoacides, et qui présente une activité biologique comparable à celle de ladite protéine.

6. Protéine capable de conférer une résistance à l'auréobasidine suivant la revendication 5, dans laquelle le 240ème résidu d'aminoacide Ala a été remplacé par Cys.

7. Protéine capable de conférer une résistance à l'auréobasidine suivant la revendication 6, représentée par la SEQ ID N° 18 ou 19 dans la liste des séquences.

8. ADN codant pour une protéine capable de conférer une résistance à l'auréobasidine suivant la revendication 5, 6 ou 7.

9. ADN suivant la revendication 8, représenté par la SEQ ID N° 20 ou 21 dans la liste des séquences.

10. Vecteur d'intégration chromosomique pour un champignon hôte suivant la revendication 1 ou 2, dans lequel le gène de résistance à l'auréobasidine est un ADN suivant la revendication 8 ou 9.

11. Procédé pour la production d'un transformant suivant la revendication 3 ou 4, dans lequel le gène de résistance à l'auréobasidine est un ADN suivant la revendication 8 ou 9.
